(19) **Europäisches Patentamt / European Patent Office / Office européen des brevets**

(11) **EP 3 821 445 B1**

(12) **FASCICULE DE BREVET EUROPEEN**

(45) Date de publication et mention de la délivrance du brevet:
**07.08.2024  Bulletin 2024/32**

(21) Numéro de dépôt: **19742703.2**

(22) Date de dépôt: **12.07.2019**

(51) Classification Internationale des Brevets (IPC):
**G16H 50/20** (2018.01)     **G16H 10/20** (2018.01)

(52) Classification Coopérative des Brevets (CPC):
**G16H 10/20; G16H 50/20**

(86) Numéro de dépôt international:
**PCT/EP2019/068848**

(87) Numéro de publication internationale:
**WO 2020/011988 (16.01.2020 Gazette 2020/03)**

(54) **SYSTEME ET PROCEDE DE GENERATION D'UNE LISTE DE PROBABILITES ASSOCIEE A UNE LISTE DE MALADIES, PRODUIT PROGRAMME D'ORDINATEUR**

**SYSTEM UND VERFAHREN ZUR ERZEUGUNG EINER LISTE VON WAHRSCHEINLICHKEITEN, DIE EINER LISTE VON KRANKHEITEN ZUGEORDNET SIND, COMPUTERPROGRAMMPRODUKT**

**SYSTEM AND METHOD FOR GENERATING A LIST OF PROBABILITIES ASSOCIATED WITH A LIST OF DISEASES, COMPUTER PROGRAM PRODUCT**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.07.2018  FR 1800757**

(43) Date de publication de la demande:
**19.05.2021  Bulletin 2021/20**

(73) Titulaire: **E-Diagmed**
**75015 Paris (FR)**

(72) Inventeurs:
• **TSOUDEROS, Yannis Emmanuel Serge**
  **92200 NEUILLY SUR SEINE (FR)**
• **GARNIER, Nicolas**
  **38400 Saint Martin d'Hères (FR)**

(74) Mandataire: **Pichon, Julien**
**Oak & Fox**
**94 rue La Fayette**
**(Esc. D)**
**75010 Paris (FR)**

(56) Documents cités:
**WO-A1-2015/122026**

• **SEIXAS FLÁVIO LUIZ ET AL: "A Bayesian network decision model for supporting the diagnosis of dementia, Alzheimer?s disease and mild cognitive impai", COMPUTERS IN BIOLOGY AND MEDICINE, NEW YORK, NY, US, vol. 51, 28 May 2014 (2014-05-28), pages 140 - 158, XP028876165, ISSN: 0010-4825, DOI: 10.1016/J.COMPBIOMED.2014.04.010**
• **ELIZABETH S BURNSIDE ET AL: "Using a Bayesian network to predict the probability and type of breast cancer represented by microcalcifications on mammography", STUDIES IN HEALTH TECHNOLOGY AND INFORMATICS, 1 January 2004 (2004-01-01), Netherlands, pages 13, XP055584305, Retrieved from the Internet <URL:https://www.ncbi.nlm.nih.gov/pmc/articles/PMC2243709/pdf/procamiasymp00003-0141.pdf> [retrieved on 20190502]**
• **MICHAEL J. PENCINA ET AL: "Predicting the 30-Year Risk of Cardiovascular Disease : The Framingham Heart Study", CIRCULATION, vol. 119, no. 24, 23 June 2009 (2009-06-23), US, pages 3078 - 3084, XP055584393, ISSN: 0009-7322, DOI: 10.1161/CIRCULATIONAHA.108.816694**

**Description**

**DOMAINE**

Le domaine de l'invention concerne les systèmes et méthodes, notamment des méthodes mises en oeuvre par ordinateur, permettant de générer une liste de probabilités conditionnelles lesquelles sont associées à des maladies. Le domaine de l'invention vise à fournir un outil, voir un simulateur, s'appuyant sur une modélisation d'un réseau Bayésien permettant à un utilisateur d'établir des diagnostics sur la base d'hypothèses formalisées. Le domaine de l'invention concerne également les méthodes visant à détecter l'influence d'un facteur dans la description d'une maladie ou dans une forme clinique particulière et identifiée de cette maladie.

**ETAT DE L'ART**

Actuellement, il existe de nombreuses méthodes permettant d'assister un médecin dans l'établissement d'un diagnostic pour un patient. Généralement ces méthodes établissent un modèle de données permettant d'associer l'apparition d'un facteur avec la survenue d'une maladie, l'association pouvant être réalisée, par exemple, par la modélisation et le calcul d'une probabilité. Le médecin peut alors définir un certain nombre de facteurs d'entrée tels que des signes, également appelés symptômes dans la terminologie médicale, afin de connaitre l'ensemble des maladies pouvant induire ce symptôme.

Un des modèles pouvant être mis en oeuvre est un modèle de type Bayésien. Un tel modèle de données permet de générer des probabilités conditionnelles en fonction d'un certain nombre de facteurs identifiés chez un patient. Un intérêt est d'établir un outil permettant d'aider un utilisateur ou un médecin à établir des diagnostics. C'est par exemple le cas de la solution décrite dans le document de brevet US7720779 dans laquelle un lien d'influence logique est modélisé.

SEIXAS FLÁVIO LUIZ ET AL: "A Bayesian network décision model for supporting the diagnosis of dementia, Alzheimer's disease and mild cognitive impai",COMPUTERS IN BIOLOGY AND MEDICINE, NEW YORK, NY, US, vol. 51, 28 mai 2014 (2014-05-28), pages 140-158, concerne une procédé pour la prédiction de probabilités d'une maladie sur la base de données d'entrée en utilisant des réseaux Bayésiens. Cet document ne divulgue pas que les signes comportent des statistiques de sensibilité et de spécificité et que le système calcule un ensemble de probabilités associés à des maladies. Cet document peut être considéré comme l'art antérieure le plus proche.

Un problème est que les liens d'influence logique peuvent conduire à écarter des possibilités ou à prendre en compte une trop large base de maladies potentielles. Cette modélisation peut conduire un utilisateur à commettre des erreurs dans l'établissement de son diagnostic. Une raison est que les signes observés peuvent différer d'un individu à l'autre, d'une forme clinique à l'autre d'une même maladie et d'une maladie à l'autre. Enfin, la modélisation d'une probabilité se fondant uniquement sur un lien logique ne permet pas de générer des listes pertinentes de probabilités associées à des maladies. En effet, le lien logique ne modélise pas, par exemple, un niveau de spécificité d'un signe dans une liste de maladies donnée pouvant l'induire.

Il existe un besoin de proposer un dispositif, et une méthode, permettant de fournir à un utilisateur un outil d'assistance pour qu'il puisse réaliser des diagnostics fiables.

**RESUME DE L'INVENTION**

Le procédé de l'invention permet de résoudre les problèmes précités.

Selon un aspect, l'invention concerne un système comportant un calculateur pour la génération d'une liste de probabilités associée à une liste de maladies pour un premier patient, ledit système comportant une interface permettant :

- une première acquisition d'un premier ensemble de premiers facteurs relatif au premier patient et enregistrement desdits premiers facteurs dans une mémoire, lesdits premiers facteurs comportant :

  ○ une valeur d'âge,
  ○ une valeur de genre ;

- une seconde acquisition d'un second ensemble de seconds facteurs, dits facteurs de risques, décrivant notamment au moins une maladie ou une information propre audit patient et un enregistrement desdits seconds facteurs dans une mémoire, ladite maladie étant extraite d'une première base de données, chaque maladie étant associée à une première statistique de prévalence et/ou d'une première statistique d'incidence, et chaque maladie étant associé à une liste de signes ;
- une troisième acquisition d'un troisième ensemble de troisièmes facteurs décrivant au moins un signe et enregistrement desdits troisièmes facteurs dans une mémoire du système, ledit premier signe comportant une première statistique de sensibilité et une seconde statistique de spécificité pour chaque maladie d'une liste prédéfinie de maladies associée audit signe ;

ledit calculateur générant, à partir d'une première modélisation d'un réseau Bayésien et de données d'entrées comportant les données de la première, seconde et de la troisième acquisition, un ensemble de probabilités, chaque probabilité étant associé à une maladie donnée de la première liste ; ledit système comportant une interface graphique pour afficher ladite première liste générée.

Un avantage est de permettre de fiabiliser des calculs de probabilités associées à des maladies grâce à un modèle intégrant une codification de la sensibilité et de la spécificité de facteurs. Un autre intérêt est de considérer différents types de facteurs, tels que le profil d'un utilisateur, c'est-à-dire d'un patient, les antécédents et les symptômes de ce dernier, dans l'évaluation des probabilités associées aux maladies.

En outre, un avantage est de construire des noeuds d'un réseau bayésien pour optimiser des calculs de probabilité en fonction d'une pluralité de facteurs.

Selon un mode de réalisation, le système comprend une interface permettant une quatrième acquisition de quatrièmes facteurs décrivant un produit médical donné et au moins un second signe associé audit produit médical donné, ledit second signe comportant une première statistique de sensibilité et une seconde statistique de spécificité pour chaque maladie d'une seconde liste prédéfinie de maladies associée audit second signe ; le calculateur générant l'ensemble de probabilités en prenant en compte en entrées les données de la quatrième acquisition pour générer la première liste.

Un avantage est de prendre en compte dans le calcul des probabilités associées à chaque maladie des données relatives à un principe actif susceptible d'interférer dans l'estimation des probabilités.

Selon un mode de réalisation, le système comporte une mémoire pour enregistrer des données décrivant au moins deux produits médicaux et enregistrer des données provenant d'une pluralité d'acquisitions de données provenant d'un ensemble de patients de telle sorte qu'un premier groupe de patients soit associé au premier produit et un second groupe de patients soit associé au second produit, le calculateur générant deux listes de probabilités conditionnelles, chaque probabilité étant associée à une maladie donnée, le calculateur effectuant un calcul pour comparer les deux listes afin de déduire la présence d'au moins un écart de probabilités pour une même maladie entre les deux listes lorsque ledit écart est supérieur à un seuil prédéfini.

Un avantage est d'identifier des effets pharmacologiques non connus d'un principe actif donné lorsqu'il est testé en regard d'un autre principe actif ou d'un placebo.

Selon un mode de réalisation, le système comprend une ressource linguistique comportant une ontologie, un dictionnaire ou une base de synonymes permettant d'associer des termes décrivant des signes ou des maladies dans la base de données à un corpus de textuel prédéfini.

Un avantage est d'homogénéiser la description des signes afin de normaliser les codifications de sensibilité et de spécificité des signes. En outre, un autre avantage est de prendre en considération des descriptions faites par des patients et traduites automatiquement. Un autre intérêt est de suggérer automatiquement par l'intermédiaire de l'interface des choix afin de mieux décrire un signe lorsqu'un signe détaillé est associée à une sensibilité ou à une spécificité prédéfinie dans une base de données maladies. Il est alors possible de générer automatiquement dans l'interface des propositions pour compléter la description d'un signe en fonction des probabilités calculées et associées à des maladies. En conséquence, l'utilisateur est amené à mieux définir les signes ou symptômes existants.

Selon un mode de réalisation, chaque troisième facteur comporte une pluralité de propriétés décrivant un signe et enregistrées dans une mémoire du système, chaque propriété étant associée à une valeur de sensibilité et de spécificité.

Selon un mode de réalisation, la première modélisation du réseau Bayésien comporte, pour au moins un noeud du réseau, une modélisation d'au moins un lien entre deux facteurs d'un des quatre ensembles de facteurs, ladite modélisation spécifiant si les facteurs sont indépendants ou dépendants et étant enregistrée dans une mémoire du système.

Un avantage est de modéliser des signes ayant un lien entre eux différemment de signes n'ayant pas de lien entre eux ou dont le lien n'est pas connu. Ainsi, l'invention permet de modéliser les noeuds du réseau bayésien selon différents niveaux de connaissance des liens causaux entre signes.

Selon un mode de réalisation :

- Lorsqu'aucun lien de dépendance entre au moins deux facteurs présents dans les données acquises n'est spécifié, le calcul de la probabilité d'une maladie comprend un calcul de probabilité conditionnelle à partir des facteurs considérés comme indépendants ;
- lorsque ladite modélisation bayésienne spécifie un lien de dépendance entre au moins deux facteurs, la probabilité d'une maladie comprend une sélection dans la base de données soit de la de probabilité conditionnelle conjointe soit une sélection d'une valeur de sensibilité et/ou de spécificité des facteurs considérés conjointement.

Un avantage est de modéliser un réseau bayésien qui soit le plus fidèle possible à la réalité. Ainsi, la modélisation des noeuds prend en compte l'existence de certains types de liens entre signes lorsqu'il existe. Dans le cas contraire, les calculs de probabilités sont réalisés en considérant les facteurs comme indépendants.

Selon un mode de réalisation, le système comprend un calculateur pour établir une comparaison entre les données acquises par l'intermédiaire de l'interface et les données enregistrées dans la mémoire de sorte que lorsqu'un facteur de risque acquis est associé à une statistique de prévalence ou d'incidence d'une maladie, la probabilité de la maladie associée est initialisée à la valeur enregistrée dans la base de données.

Selon un autre aspect l'invention concerne un procédé de génération d'une liste de probabilités associée à une liste de maladies pour un premier patient, ledit procédé comportant :

- Première acquisition d'un premier ensemble de premiers facteurs relatif au premier patient comportant :

  ○ une valeur d'âge,
  ○ une valeur de genre ;

- Seconde acquisition d'un second ensemble de seconds facteurs, dits facteurs de risques, décrivant notamment au moins une maladie ou une information propre audit patient dudit patient, ladite maladie étant extraite d'une première base de données, chaque maladie étant associée à une première statistique de prévalence et d'une première statistique d'incidence, et chaque maladie étant associée à une liste de signes ;
- Troisième acquisition d'un troisième ensemble de troisièmes facteurs décrivant au moins un signe, ledit premier signe comportant une première statistique de sensibilité et une seconde statistique de spécificité pour chaque maladie d'une liste prédéfinie de maladies associée audit signe ;
- Application d'une première modélisation d'un réseau Bayésien à des données d'entrées comportant les données de la première, seconde et de la troisième acquisition, pour la génération d'un ensemble de probabilités, chaque probabilité étant associé à une maladie donnée de la première liste. Un avantage est d'implémenter le procédé de tel sorte qu'il puisse accéder

à une base de données de signes et ou de maladies de manière à calculer à partir de données collectées par une interface une liste de maladies, chacune étant associée à une probabilité. Un avantage est de prendre en compte un profil donné d'un utilisateur et des données propres à cet utilisateur pour fiabiliser les données de probabilité qui sont générées.

Selon un mode de réalisation, la probabilité associée à une maladie de la première liste est une probabilité conditionnelle d'une maladie à la survenance d'un ensemble de facteurs comportant au moins trois éléments parmi la liste suivante :

- un signe prédéfini ;
- un antécédent prédéfini ;
- un facteur de risque ;
- un âge prédéfini ;
- un genre prédéfini ;
- un lieu géographique prédéfini ;

Un avantage est de prendre en compte de nombreux facteurs pour améliorer la précision des probabilités qui sont générées dans chaque liste.

Selon un mode de réalisation, au moins un facteur de la seconde acquisition de données est associé à un antécédent et comporte au moins un des critères suivants :

- une première date d'apparition ;
- une fréquence d'apparition ;
- une information génétique.

Un avantage est de sélectionner, le cas échéant, une statistique de prévalence ou d'incidence dans la base de données de signes ou de maladies qui est propre à un second facteur correspondant à un antécédent qualifié par au moins une donnée supplémentaire. Ainsi, la base de données de signes ou de maladies comporte une pluralité de statistiques affectées à différentes qualifications d'antécédents.

Selon un mode de réalisation, la première modélisation du réseau Bayésien comporte, pour au moins un noeud du réseau, une modélisation d'au moins un lien entre deux facteurs d'un des quatre ensembles de facteurs, ladite modélisation spécifiant si les facteurs sont indépendants ou dépendant, la relation de dépendance entre au moins deux facteurs étant modélisée par une valeur de probabilité conditionnelle conjointe d'une maladie sachant les facteurs présents.

Selon un mode de réalisation, le procédé comprend une étape de vérification de l'existence d'un lien entre facteurs acquis des différents ensembles dans la base de données des signes ou des maladies, le cas échéant, le procédé comprend une étape de sélection de la valeur de la probabilité conjointe associée aux facteurs liés.

Un avantage est de modéliser un réseau bayésien tenant compte d'un niveau de détail de la base de données de signes ou de maladies. Le niveau de détail est défini par la définition de statistiques de prévalence ou d'incidence ou de spécificité de signes pour une maladie et de sensibilité de signes dans une maladie considérés conjointement avec d'autres facteurs. Lorsqu'une nouvelle statistique, ou probabilité, est définie, le réseau bayésien est modélisé de telle sorte que ses noeuds prennent en compte les liens entre facteurs affectés par cette nouvelle statistique. Ainsi selon les données acquises d'un utilisateur, les calculs de probabilités sont améliorés.

Selon un mode de réalisation, le procédé comprend une quatrième acquisition de quatrièmes facteurs décrivant un produit médical donné et au moins un second signe associé audit produit médical donné, ledit second signe comportant une première statistique de sensibilité et une seconde statistique de spécificité pour chaque maladie d'une seconde liste prédéfinie de maladies associée audit second signe ; l'étape de génération de l'ensemble de probabilités prenant en compte en entrées les données de la quatrième acquisition.

Un avantage est de permettre d'identifier des probabilités associées à des maladies pouvant être affecté notamment à la présence d'un quatrième facteur.

Selon un mode de réalisation, la sensibilité ou la spécificité d'un signe d'une entrée de la première base comporte :

- Soit une valeur exprimant une probabilité ;
- soit une valeur sélectionnée d'une échelle discrète prédéfinie, ladite échelle discrète prédéfinie associant à chacune de ses valeurs une probabilité par groupe d'âge et/ou de genre.

Un avantage est de normaliser les valeurs de sensibilité et de spécificité de sorte à obtenir des calculs homogènes et pouvant être quantifier par un « dire d'expert ». Ainsi un dire d'expert pourra quantifier la sensibilité sur une échelle, par exemple comprenant entre 5 et 8 niveaux pour quantifier les valeurs, alors qu'il ne pourra pas quantifier précisément une statistique au pourcentage près, par exemple de sensibilité d'un signe. Ainsi, cette modélisation permet d'acquérir une information ayant une granularité pouvant être donnée par un expert.

Selon un mode de réalisation, le procédé est réalisé pour une pluralité de patients, le procédé comportant une pluralité d'acquisitions de données de telle sorte que chaque patient est associé à un premier produit ou à un second produit, un premier groupe de patients étant associé au premier produit et un second groupe de patients étant associé au second produit, l'étape de génération comportant la génération de deux listes de probabilités conditionnelles, chaque probabilité étant associée à une maladie donnée, le procédé comportant, en outre, une étape de comparaison des deux listes afin de déduire la présence d'au moins un écart de probabilités associées à une même maladie et dont l'écart est supérieur à un seuil prédéfini.

Un avantage est de permettre d'utiliser l'invention pour des applications de pharmacovigilances.

Selon un mode de réalisation, la troisième acquisition est réalisée au moyen d'une interface dans laquelle la sélection d'un signe donné entraine automatiquement la génération d'une première sélection de signes, lesdits signes sélectionnés étant associés avec le signe donné dans au moins une maladie, ladite interface comportant un menu affichant ladite sélection de signes.

Un avantage est de tirer profit des liens établis entre les différents facteurs lorsqu'ils existent. Cela permet de suggérer automatiquement par le biais de l'interface des choix dans la définition des données saisies par un utilisateur. Les choix proposés sont ceux qui sont le plus susceptibles d'être vérifiés par la présence et la quantification des liens entre facteurs. Pour cela les liens entre facteurs qui sont modélisés par une statistique/probabilité peuvent être proposés, à un utilisateur, selon un certain ordre selon les valeurs de probabilités.

Selon un mode de réalisation, la troisième acquisition entraine la génération d'une seconde sélection de tests, lesdits tests comportant un descriptif visant à identifier la présence d'au moins un signe chez le patient.

Selon un autre aspect, l'invention concerne un produit programme d'ordinateur chargeable directement dans la mémoire interne d'un ordinateur numérique, comprenant des portions de code de logiciel pour l'exécution des étapes du procédé de l'invention lorsque ledit programme est exécuté sur un ordinateur.

Selon un autre aspect, l'invention concerne un produit programme d'ordinateur enregistré sur un support utilisable dans un ordinateur, comprenant au moins un calculateur et une mémoire afin d'exécuter une commande pour la mise en oeuvre du procédé de l'invention.

**BREVE DESCRIPTION DES FIGURES**

D'autres caractéristiques et avantages de l'invention ressortiront à la lecture de la description détaillée qui suit, en référence aux figures annexées, qui illustrent :

- figure 1 : une architecture générale pour la mise en oeuvre du procédé de l'invention et /ou du système de l'invention ;
- figure 2 : une ontologie de signes selon leur manifestation et leur description ;
- figure 3 : un schéma des différents facteurs étant pris en compte dans le calcul d'une probabilité de la survenue d'une maladie selon un mode de réalisation du système de l'invention.

**DESCRIPTION**

**Définitions**

La sensibilité d'un test mesure sa capacité à donner un résultat positif lorsqu'une hypothèse est vérifiée. Dans le cas de l'invention, la sensibilité d'un signe mesure sa possibilité d'être présent dans la manifestation d'une maladie. La sensibilité peut être exprimée en pourcentage ou en ratio. Selon un mode de réalisation de l'invention, le niveau de détails qualifiant la description d'un signe permet d'améliorer la pertinence de la sensibilité dudit signe.

La spécificité mesure la capacité d'un test à donner un résultat négatif lorsque l'hypothèse n'est pas vérifiée. Dans le cadre de l'invention, la spécificité d'un signe est sa capacité à prédire la non-présence de la maladie lorsqu'il n'est pas présent. Il peut également être exprimé comme la mesure du caractère spécifique d'un signe par rapport à une maladie. Il peut être exprimé en pourcentage, c'est-à-dire une mesure indiquant 90% de spécificité d'un signe pour une maladie indique une forte caractérisation de la présence de la maladie lorsque le signe est détecté. Selon un mode de réalisation de l'invention, le renseignement d'une description détaillée et précise d'un symptôme/ signe permet de modifier la valeur de la spécificité. Le niveau de précision et de détail d'un signe peut être associé à une échelle de valeurs de la spécificité d'un signe pour une maladie.

Un signe peut avoir une sensibilité faible dans une maladie et une forte spécificité pour la même maladie, ou inversement, une forte sensibilité et peu de spécificité.

Une association de signes peut avoir une sensibilité plus faible dans une maladie et une spécificité plus forte pour la même maladie, respectivement, que celles-ci pour chacun de ces signes isolés.

La prévalence $PR_i$ est une mesure de l'état de santé d'une population, dénombrant le nombre ou une proportion de cas d'une maladie à un instant donné ou sur une période donnée. La prévalence peut être associée à un antécédent ou autrement dit à un facteur de risque. La prévalence peut également être associée à une démographie donnée. La prise en compte dans la définition de la démographie d'une tranche d'âge, d'un genre, d'antécédents familiaux et/ou personnels, ou d'une zone géographique ou d'une combinaison de ces critères peut être associée à une prévalence donnée. Dans le cadre de l'invention, un modèle de prévalence comprend les différentes valeurs de prévalence sur une segmentation donnée d'une démographie et/ou de facteurs de risques.

L'incidence $INi$ d'une maladie mesure le nombre de cas apparus pendant une durée prédéfinie, par exemple une année, au sein d'une population. L'incidence d'une maladie peut être associée à une démographie donnée. La prise en compte dans la définition de la démographie d'une tranche d'âge, d'un genre ou d'une zone géographique ou d'une combinaison de ces critères peut être associée à une incidence donnée de la maladie. Dans le cadre de l'invention, un modèle d'incidence d'une maladie comprend les différentes valeurs d'incidence de la maladie sur une segmentation démographique donnée et/ou de facteurs de risques.

Une maladie $M_i$ est une altération ou un trouble de l'organisme. Dans le cadre de l'invention, une base de données de maladies $BD_M$ est utilisée. La base de données des maladies $BD_M$ comporte des champs dont les valeurs caractérisent la maladie et des champs permettant de définir des indicateurs contextuels relatifs à sa survenance, tels que son incidence ou sa prévalence.

Un symptôme Si est un signe clinique qui résulte d'une manifestation d'une maladie, tel qu'exprimé et ressenti par un patient. Dans l'invention, chaque signe est décrit dans une base de données. Un symptôme correspond à la description qu'un patient donne d'un signe. Ainsi, les termes et l'ontologie peuvent différer entre la description faite par un patient et celle de la base de données de signes.

Dans le cadre de l'invention, une base de données de signes BDs est donc définie. Selon un mode de réalisation, un dictionnaire, une base de synonymes ou encore une ontologie peuvent être intégrés au système et à la méthode de l'invention. Une conséquence est de permettre de détecter un signe ou une propriété d'un signe lorsque certains termes sont saisis dans l'interface par un utilisateur. Ainsi, un utilisateur peut être guidé par des suggestions, des définitions ou des explications permettant d'interpréter la description d'un symptôme pour lui associer des propriétés d'un signe.

Une maladie donnée peut avoir des tableaux cliniques différents à l'instant T par rapport à T+dt et/ou d'un

patient $P_i$ à l'autre patient $P_k$. La base de données des signes BDs comporte des champs dont les valeurs caractérisent ledit signe. En outre, elle comporte des champs permettant de définir des indicateurs contextuels relatifs aux liens entre le signe et des maladies ou encore des liens entre des signes entre eux. Ces liens permettent de prendre en compte des pondérations de valeurs des facteurs définissant des données d'entrées dans le calcul d'une probabilité pour une maladie donnée. Pour chaque signe $S_k$, une liste de maladies $LIST_{Sk}$ est associée avec, pour chacune d'entre elles, une valeur de spécificité et une valeur de sensibilité.

La base de données des maladies $BD_M$ et celle des signes BDs sont donc liées notamment par les spécificités $SP_i$ pour une ou plusieurs maladie(s) et les sensibilités SEi de signes dans une ou plusieurs maladies.

La base de données des maladies $BD_M$ et/ou celle des signes BDs peut/vent donc être complétée(s) par un champ relatif à au moins un signe Si associé à une maladie donnée avec les valeurs de sensibilité et de spécificité dudit signe Si. En décrivant un symptôme d'un patient, il est alors possible de sélectionner un signe $S_k$ déjà renseigné dans la base de données. Une interface permet alors à un utilisateur de déterminer les caractéristiques du symptôme afin de filtrer les résultats possibles présents dans la base de données.

Un « antécédent » est par exemple une maladie qui a été ou qui est déclarée par un patient. Plus généralement, dans le cadre de la présente invention, un antécédent est un fait propre à un patient induisant un facteur de risque d'une maladie. À titre d'exemple, une maladie antérieure, un statut de fumeur, un cholestérol supérieur à un seuil donné, une mutation génétique, une prise médicamenteuse sont des faits rattachés à un patient qui peuvent être considérés comme des facteurs de risque pour un ensemble de maladies.

Elle correspond à une maladie contractée par le patient. L'invention permet de prendre en considération dans le profil d'un patient un ensemble de facteurs, dont les antécédents. Ces derniers peuvent affecter les probabilités associées à une maladie dans la mesure où ces derniers peuvent être par exemple spécifiques d'une maladie.

Cela peut être le cas, par exemple, dans la prise en compte de l'antécédent : « Cirrhose du foie » lors du calcul de la probabilité de la maladie : « Encéphalopathie hépatique ». En effet, l'antécédent « Cirrhose du foie » affecte ici de manière conséquente la probabilité de la maladie « Encéphalopathie hépatique » du fait :

- de la valeur de sensibilité de « Cirrhose du foie » dans l'« Encéphalopathie hépatique » et ;
- de la valeur de spécificité de la « Cirrhose du foie » pour l'« Encéphalopathie hépatique ».

**Modélisation de la sensibilité**

Selon un mode de réalisation, un champ de sensibilité d'un signe relativement à une maladie est codé selon une échelle prédéfinie de valeurs, par exemple de 0 à 6. « 0 » correspondant au plus à 5% en sensibilité d'une maladie et « 6 » à au moins 95% en sensibilité d'une maladie, entre 1 et 5, les quotients ou les pourcentages de sensibilité à une maladie sont déterminés selon une distribution de probabilités qui est prédéfinie. La distribution peut être, par exemple, de type Gaussienne ou linéaire. Toute autre courbe d'association est compatible avec l'invention. Un modèle linéaire peut, par exemple, être mis en oeuvre. Selon un autre exemple, l'échelle de valeurs est établie entre 0 et 10. L'invention est compatible avec toute autre mise en oeuvre d'échelle de valeurs.

Lorsque la valeur de la sensibilité d'un signe dans une maladie est connue pour un âge, un genre, ou un antécédent ou un facteur externe pathogène donné, le procédé de l'invention permet de prendre en compte la valeur connue dans la base de données $BD_M$ ou BDS. Cette donnée peut permettre de renforcer un modèle de distribution de valeurs de sensibilité pour une population donnée.

De la même manière, la sensibilité d'un antécédent dans une maladie est enregistrée dans la base de données du système de l'invention. Selon un mode de réalisation de l'invention, l'acquisition d'un antécédent ANT1 décrit dans le profil d'un patient P1 est prise en compte dans la détermination de la probabilité d'une maladie P(M) lorsque la valeur de la probabilité de la maladie associée à l'antécédent est spécifiée dans la base. L'invention permet donc de modéliser les liens entre antécédents et maladies par la définition d'un ensemble de probabilités. Cette probabilité peut être interprétée comme la sensibilité d'un antécédent dans une maladie.

**Modélisation de la spécificité**

Selon un mode de réalisation, un champ de spécificité d'un signe $S_k$ pour une maladie est codé selon une échelle prédéfinie de valeurs, par exemple de 0 à 6. Selon un autre exemple, l'échelle de valeurs est établie entre 0 et 10. L'invention est compatible avec toute autre mise en oeuvre d'échelle de valeurs. De manière analogue à la modélisation de la sensibilité, les valeurs de l'échelle choisie pour modéliser la spécificité peuvent reposer sur une distribution discrète de probabilités pour une population donnée. Par exemple, si l'échelle est comprise entre 0 à 6, « 0 » correspondant au plus à 5% en spécificité d'un signe pour une maladie et « 6 » au moins à 95% en spécificité d'un signe pour une maladie. Entre 1 et 5, les pourcentages de spécificités sont associés aux valeurs de l'échelle selon une distribution de probabilités pour une population donnée. La distribution peut être, par exemple, de type Gaussienne ou linéaire.

Toute autre courbe d'association est compatible dans l'invention.

Lorsque la valeur de la spécificité est connue, le procédé de l'invention permet de prendre en compte la valeur connue dans la base de données $BD_M$. En cas de conflit entre une échelle prédéfinie et une valeur donnée, le procédé de l'invention prend en compte la valeur connue enregistrée dans la base de données. Une alarme peut être générée lors de la détection d'un cas de conflit afin d'attirer l'attention de l'utilisateur ou de tout autre personnel sur la modélisation de la spécificité d'un signe d'une maladie donnée.

De la même manière, la spécificité de la présence d'un antécédent pour une maladie est enregistrée dans la base de données du système de l'invention. D'un point de vue de l'invention, la précision d'un antécédent précisé dans le profil d'un patient agit de la même manière que précision d'un antécédent. L'invention permet donc de modéliser les liens entre antécédents et maladies par la définition d'un ensemble de probabilités de la spécificité d'un antécédent relativement à au moins une maladie.

**Signe détaillé**

Un « signe détaillé » est également appelé un « signe qualifié ».

Le procédé comprend un moyen de calcul automatique de la valeur de la spécificité d'un « signe détaillé » pour une ou plusieurs maladies en fonction des données renseignées dans les différents champs de sa description. On rappelle qu'un « signe détaillé » comprend plus d'informations qu'un « signe générique ». Ce calcul automatique peut, par exemple, reposer sur un abaque ou une échelle prédéfinie permettant de quantifier le niveau de précision de la description d'un signe et les valeurs de la spécificité et de la sensibilité qui sont associées à chaque gradient de ladite échelle ou de l'abaque prédéfini.

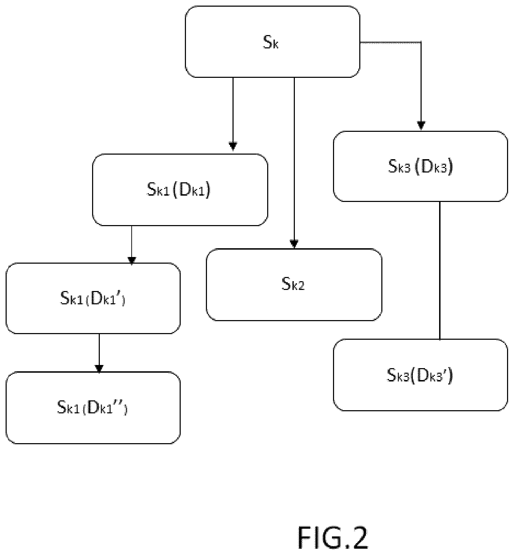

La figure 2 représente un signe générique $S_k$ et des manifestations différentes de ce signe, notées $S_{k1}$, $S_{k2}$, $S_{k3}$. Le signe $S_{k1}$ est décliné selon des descriptions plus ou moins détaillées : $D_{k1}$, $D_{k1}'$, $D_{k1}"$. Dans cet exemple, la description $D_{k1}"$ est plus détaillée que la description $D_{k1}'$ qui est, elle-même, plus détaillée que la description $D_{k1}$. La raison de ces écarts peut provenir d'une acquisition différente des données ou de nuances dans les manifestations de ces signes selon les patients et plus encore selon les maladies.

Selon un mode de réalisation, la spécificité $SP_k$ est codée sur une échelle prédéfinie qui permet de générer un pourcentage associé à la valeur correspondante lors de la modélisation du réseau Bayésien RB mis en oeuvre. La valeur de la spécificité $SP_k$ peut être prédéfinie pour chacune des déclinaisons/ manifestations d'un signe $S_k$ et de chaque type de description $D_{ki}$, $D_{ki}'$, $D_{ki}"$ de ce dernier pour une ou plusieurs maladie(s). Les valeurs sont enregistrées dans une mémoire.

Selon un mode de réalisation, la spécificité $SP_k$ d'un « signe détaillé » $S_{ki}$ pour une maladie donnée peut être obtenue en considérant une spécificité générale du signe et en appliquant un coefficient de pondération obtenu en fonction du nombre de champs de la description. Il s'agit alors d'une pondération quantitative dont le principe repose sur le fait que plus un signe $S_k$ est détaillé plus il est spécifique.

Selon un autre exemple qui peut être combiné à ce dernier, la valeur des champs permet de générer un coefficient de pondération de la spécificité d'un signe $S_k$ pour une ou plusieurs maladie(s). Dans ce cas, il s'agit d'une pondération qualitative dont le principe repose sur la prise en compte des valeurs spécifiant une description d'un signe $S_k$.

Selon un mode de réalisation, la valeur de la sensibilité $SE_k$ d'un signe $S_k$ pour une maladie donnée $M_1$ est codée de la même manière que la spécificité $SP_k$. Il est de même pour une « sensibilité détaillée » ou une « sensibilité générique ».

Chaque signe $S_k$ comporte une valeur de spécificité pour une ou plusieurs maladie(s) qui peut être ajustée selon la description détaillée $S_{ki}$ d'une manifestation de ce signe. Lorsque la description d'un signe $S_{ki}$ est modifiée, selon un mode de réalisation de l'invention, une interface est générée permettant à un utilisateur de modifier les valeurs de spécificité. Par défaut, sans modification de l'utilisateur, la valeur de la spécificité reste inchangée dans la base de données.

Selon un exemple, les valeurs de spécificité et de sensibilité des signes suivants sont renseignées dans la base de données :

- le signe : « douleur » comprend une spécificité de Sp = 0,0% et une sensibilité Se = 100%.
- le signe qualifié : « douleur thoracique » comprend une spécificité de Sp = $x_1$ > 0.0% et une sensibilité Se = $y_1$ < 100%.
- le signe qualifié « douleur antérieure » comprend une spécificité de Sp= $x_2$ > $x_1$ et une sensibilité Se = $y_2$ < $y_1$.
- le signe qualifié « douleur thoracique antérieure » comprend une spécificité de Sp = $x_3$ > $x_1$ et une sensibilité Se = $y_3$ < $y_1$.

La sensibilité Se et la spécificité Sp sont définies et modifiées par exemple par un utilisateur ou plusieurs utilisateurs ayant des droits spécifiques leur permettant d'accéder à ces données et de les modifier dans la base, ils sont appelés « administrateur ». Selon un mode de réalisation, pour un utilisateur utilisant le procédé ou le système de l'invention, les valeurs de sensibilité et de spécificité sont déterminées et fixées lors de l'utilisation du logiciel mettant en oeuvre le procédé ou le système de l'invention.

Selon un mode de réalisation, les valeurs sont modifiées par exemple à partir d'une mise à jour opérée par un administrateur de la ou des base(s) de données. Selon un autre mode de réalisation, les bases de données du système peuvent être automatiquement mises à jour avec une base de données centralisant les valeurs à jour des différents paramètres.

On rappelle qu'un signe Si plus détaillé sera plus précis et donc moins fréquent, mais par ailleurs plus spécifique.

Un intérêt d'une modélisation d'un signe détaillé est d'associer à chaque caractéristique du signe une valeur de sensibilité et de spécificité lorsque ces dernières sont connues.

## Modélisation d'un signe

Selon un mode de réalisation, une interface permet de détailler la description $D_{ki}$ d'un signe détaillé $S_{ki}$, appelé « signe détaillé », d'un patient. Le signe détaillé $S_{ki}$ comprend au moins la description d'un signe générique $S_k$ comportant lui-même au moins une désignation du signe, par exemple : « la fièvre ».

En outre, selon différents exemples, la description d'un signe détaillé comprend différents champs tels que :

- une intensité du signe, par exemple définie sur une échelle prédéfinie ou selon des qualificatifs prédéfinis
- une courbe d'occurrence du signe, par exemple, restituant un nombre de manifestations du signe ou une évolution de son apparition ;
- une qualification du signe comme, par exemple, une toux sèche ou une toux grasse ;
- une valeur restituant la persistance et l'évolution du signe, spontanément ou dans la durée d'un traitement ou après un changement de posologie ou de la prise d'un médicament ;
- un contexte de manifestation du signe, par exemple en fonction de l'heure, du jour ou encore en fonction d'une condition climatique ou géographique ;
- un résultat d'examen médical (test radiologique, scanner, biologie, palpations, etc.) ;
- etc.

Plus une description $D_{ki}$ d'un signe détaillé $S_{ki}$ est renseignée dans la base de données $BD_M$ ou la base DBs, plus elle permet d'obtenir des probabilités élevées pour une maladie donnée ou un ensemble de maladies donné.

En effet, chaque propriété d'un signe peut être modélisée par une valeur de spécificité et de sensibilité.

Pour chaque signe décrit, l'invention permet d'en qualifier précisément la description au moyen d'une interface enrichie. Un menu permet pour ce signe de choisir parmi les différents qualificatifs qui lui sont possiblement liés dans les différentes maladies où il peut exister. Lors d'une nouvelle entrée, par exemple lorsqu'un nouveau patient décrit un signe pour la première fois, le procédé de l'invention permet : soit d'enrichir un signe détaillé existant, soit de définir un nouveau signe détaillé. Dans ce dernier cas, un menu permet de sélectionner un signe existant et d'en éditer une nouvelle version afin de compléter ce dernier avec des informations supplémentaires collectées, par exemple, par un utilisateur.

Ici est présenté dans un tableau, un exemple de modélisation provenant d'un extrait d'une description de signes liés à la confusion dans la maladie encéphalopathie hépatique :

| Signes | Qualificatifs | Sensibilité | Spécificité |
|---|---|---|---|
| Survenue | | | |
| | Très lente | 00 | 0 |
| | Rapide | 4 | 3 |
| | Brutale | 2 | 3 |
| | | | |
| Troubles | Fixes | 00 | 0 |
| | Fluctuants | 6 | 5 |

Les valeurs, ici indiquées sur une échelle de 1 à 6, permettent de coder une fraction/ un pourcentage qui permet de calculer une probabilité conditionnelle d'une maladie lorsque le signe est présent. Le code de sensibilité 00 permet d'écarter la maladie lorsque le code est présent, c'est-à-dire lorsque le signe ou le signe détaillé est présent. Par exemple, cela peut être le cas lors d'un résultat d'examen normal qui de fait élimine la possibilité d'une maladie donnée.

Dans cet exemple, la survenue du signe peut être associée à une valeur de spécificité pour une maladie donnée et/ou une valeur de sensibilité dans une maladie. De manière analogue, la modélisation des troubles et de leur apparition comprend l'association d'une valeur de spécificité et de sensibilité à chaque attribut présent dans le modèle, ici : « fixes » et « fluctuants ».

Un même signe générique $S_k$ peut être associé à une liste de maladies $M_{p,p\varepsilon[1,N]}$ différentes. Ce signe générique $S_k$, peut comprendre différentes présentations $S_{k1}$, $S_{k2}$, $S_{ki}$, etc., pour différentes maladies de la liste de maladies $M_{p,p\varepsilon[1,N]}$. La caractérisation de chaque signe détaillé est associée à une maladie et renforce la spécificité des signes détaillés Ski. L'invention permet donc de rattacher un concept commun, c'est-à-dire la désignation du signe, à différentes maladies tout en différenciant les champs caractérisant les manifestations du signe pour chacune d'entre elles, c'est-à-dire les signes détaillés.

De la même manière, chaque maladie $M_p$ est associée à une liste de signes $S_{ki}$ de telle sorte que les bases des signes BDs et des maladies $BD_M$ sont associées entre elles.

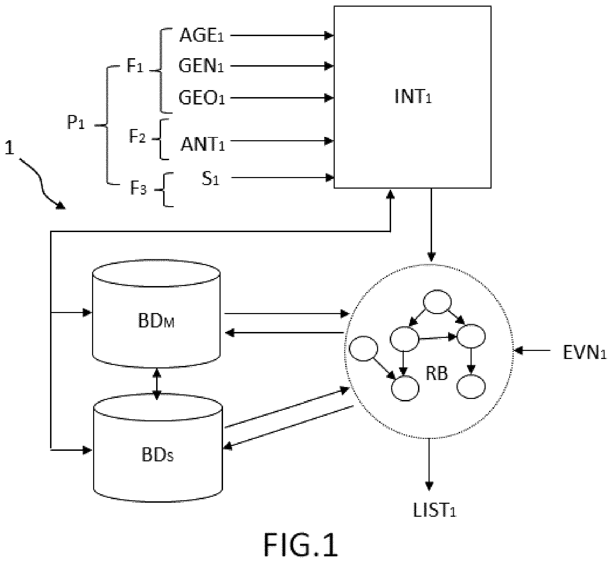

La figure 1 représente un mode de réalisation de l'invention. Une interface utilisateur $INT_1$ permet d'accéder aux fonctionnalités mises en oeuvre par l'exécution du procédé de l'invention. L'interface $INT_1$ peut être accessible depuis un téléphone intelligent, un ordinateur ou une tablette numérique ou encore toute autre machine comportant une mémoire et un calculateur. Elle comprend des menus, tels que des menus déroulants, et des champs de saisie permettant de définir ou sélectionner des informations. Avantageusement, l'interface $INT_1$ offre un moyen d'acquérir des données.

En outre, l'interface $INT_1$ permet d'accéder à un serveur distant ou une mémoire locale comportant une base de données de maladies $BD_M$ et/ou une base de données de signes BDs.

Le procédé de l'invention comporte une première étape d'acquisition de données $ACQ_1$ d'un patient $P_1$. Les données comprennent un identifiant afin d'identifier le patient $P_1$ de manière unique. En outre, les données définissant le patient $P_1$ peuvent comprendre une information le désignant, tel qu'un identifiant ou une donnée chiffrée pouvant être décodée par un moyen de chiffrement/ déchiffrement. Selon un mode de réalisation, les données identifiées comme confidentielles sont cryptées.

## Génération de test/examen

La manière de caractériser un signe peut être réalisée en trois phases successives comportant des étapes différentes réalisées avec un patient $P_1$ :

- description d'un signe décrit par le patient (symptôme) ;
- réalisation d'examen(s) clinique(s),
- réalisation d'examen(s) complémentaire(s), par exemple des examens biologiques ou d'imagerie.

Le procédé de l'invention permet de générer automatiquement, à l'issue de la génération d'une liste de probabilités, un indicateur mentionnant un type de test à réaliser et éventuellement sa nature. Selon un mode de réalisation, lorsque deux probabilités associées à des maladies distinctes sont sensiblement proches ou lorsque l'une d'elles est supérieure à un seuil prédéfini, un test est automatiquement proposé, quelle que soit la valeur des autres.

À titre d'exemple, si une première liste $LIST_1$ comporte trois maladies $M_1$, $M_2$, $M_3$ associées chacune aux probabilités suivantes : 30%, 25%, 2%, il est alors nécessaire de déterminer un test spécifique des maladies $M_1$ et $M_2$. L'invention permet alors d'identifier automatiquement un signe spécifique propre à l'une des deux maladies $M_1$ ou $M_2$ et qui soit discriminant de chacune des deux maladies $M_1$ et $M_2$. L'invention permet alors de générer automatiquement un indicateur précisant un test ou un examen permettant de discriminer $M_1$ et $M_2$ selon la présence ou non du signe qu'une interface du système de l'invention suggère de renseigner. À cet effet une base de données de tests/examens peut associer des protocoles de tests à des signes et/ou des maladies. Dans cet exemple, l'écart entre 25% et 30% est inférieur à un seuil prédéfini, par exemple 8%. À cette première condition, le procédé comporte une étape visant à identifier pour chacune de ces maladies, au moins un signe dont la spécificité est inférieure à un certain seuil pour l'une des deux maladies et supérieure à un certain autre seuil pour l'autre maladie. Le test permettant de déterminer et vérifier la présence du signe aidera alors un utilisateur pour conclure à un diagnostic robuste.

Selon un autre exemple, si une première liste $LIST_1$ comporte trois maladies $M_1$, $M_2$, $M_3$ associées chacune aux probabilités suivantes : 20%, 3%, 90%, il est alors nécessaire de déterminer un test spécifique des maladies $M_1$ et $M_3$. Même si la valeur de la probabilité de $M_3$ est très élevée, l'invention permet de recommander automatiquement la réalisation d'un test supplémentaire pour écarter la possibilité de $M_1$. Le système de l'invention comporte une base de données de tests référencés et décrits, chaque test étant associé à une spécificité pour une ou plusieurs maladie(s). Ainsi, chacune des maladies présentées dans la liste $LIST_1$ peut être associée à un ou plusieurs tests permettant d'améliorer le calcul des probabilités d'au moins une maladie de la liste $LIST_1$.

L'invention permet alors d'identifier automatiquement un signe spécifique propre à l'une ou à l'autre des maladies

$M_1$ ou $M_3$ et qui soit discriminant de chacune des deux maladies $M_1$ et $M_3$. L'invention permet alors de générer automatiquement un indicateur précisant un test ou un examen permettant de discriminer la présence ou non du signe. À cet effet, une base de données de tests/examens peut associer des protocoles de tests à des signes et/ou des maladies. Dans cet exemple, le seuil de 15% est franchi par deux maladies : $M_1$ et $M_3$. Dans cette seconde condition, le procédé comporte une étape visant à identifier un signe dont la spécificité est inférieure à un certain seuil, voire rédhibitoire, pour l'une des deux maladies et supérieure à un certain autre seuil pour l'autre maladie. Le test permettant de déterminer la présence du signe aidera alors un utilisateur à obtenir des probabilités plus fiables dans la liste $LIST_1$.

**Premiers facteurs F1 : les données de patients**

Les données acquises lors de l'acquisition $ACQ_1$ sont dénommées « premiers facteurs $F_1$ ». Elles comprennent au moins un âge $AGE_1$ et un genre $GEN_1$. Ces informations sont notamment prises en compte pour sélectionner automatiquement une prévalence (ou une incidence annuelle) d'une maladie associée au profil du patient $P_1$. À cette fin, la prévalence est déterminée à partir d'un modèle de prévalence selon une population donnée en rapport avec le profil du patient, dont le genre et l'âge. Par exemple, les valeurs de prévalence peuvent être distribuées selon des classes d'âges ou de genre. Selon un exemple, un menu propose des champs prédéfinis permettant de sélectionner des informations propres au patient P1 afin de générer automatiquement les données du modèle qui lui seront associées.

Selon un mode de réalisation, les données acquises d'un patient $P_1$ comprennent une information géographique $GEO_1$, par exemple un pays, une région ou encore une ville. Cette donnée peut également être prise en compte dans la détermination d'une prévalence d'une maladie. Les valeurs de prévalence d'une maladie peuvent être distribuées selon des zones géographiques. Selon un mode de réalisation, une zone de saisie permet de définir l'information géographique. Selon un autre mode de réalisation, l'information géographique $GEO_1$ est sélectionnée depuis un menu permettant d'extraire des données géographiques qui ont été prédéfinies dans une mémoire locale ou distante.

Selon un mode de réalisation, l'interface $INT_1$ permet de sélectionner au moins un antécédent $ANT_1$, c'est-à-dire une maladie $M_1$ d'un patient $P_1$ qui est survenue à une date antérieure ou actuelle. Ces données sont acquises lors d'une acquisition de données $ACQ_2$. Cette acquisition $ACQ_2$ peut être réalisée à partir de la même interface $INT_1$ que l'interface ayant permis l'acquisition des premiers facteurs $F_1$. Selon un autre mode de réalisation, une seconde interface $INT_2$ peut être utilisée, par exemple, succédant à la première interface $INT_1$ après avoir validé les données acquises par cette première interface $INT_1$. Les données représentant les seconds facteurs $F_2$ peuvent donc être enregistrées conjointement ou successivement aux premiers facteurs $F_1$, c'est-à-dire dans une même étape ou dans une étape successive.

**Seconds facteurs F₂ : les antécédents ANT**

Considérons dans cet exemple que l'antécédent $ANT_1$, c'est-à-dire une maladie, est sélectionné depuis l'interface $INT_1$ auprès de la base de données de maladies des maladies $BD_M$. Les informations sélectionnées et extraites de la base $BD_M$ sont alors associées aux informations dudit patient $P_1$. Selon un mode de réalisation, afin d'associer au moins un antécédent $ANT_1$ à un patient $P_1$, une maladie est sélectionnée. Les champs de description de la maladie $ANT_1$ peuvent être modifiés lorsqu'une valeur par défaut existe ou directement définis dans l'interface $INT_1$. À titre d'exemple, la périodicité d'apparition d'un signe associé à la maladie sélectionnée peut être renseignée, tout comme la durée et la date de la maladie. Lorsque l'antécédent $ANT_1$ est correctement défini, l'information peut être enregistrée et associée à l'identifiant dudit patient $P_1$.

Selon un mode de réalisation, une pluralité de seconds facteurs $F_2$ est définie pour un même patient $P_1$. Ainsi, un patient peut avoir plusieurs antécédents.

Selon l'invention, les seconds facteurs $F_2$ sont donc pris en compte dans un modèle bayésien afin de calculer des probabilités conditionnelles de maladies sur la base de l'existence ou non de cet antécédent $ANT_1$.

Selon un mode de réalisation, l'invention permet de coder un champ d'un antécédent permettant d'exclure la présence d'au moins une maladie donnée. Une propriété d'un antécédent $ANT_1$ peut alors être « immunisante » d'une ou plusieurs maladies. Lorsque le profil d'un patient comprend le renseignement de cet antécédent $ANT_1$ comportant ledit champ, la probabilité associée à la maladie exclue est alors de 0. Cette propriété est codée par un champ prédéfini. Le champ par défaut est configuré par exemple sur la valeur « non immunisante ». Le champ d'exclusion peut également être codé lorsqu'un signe est présent dans le renseignement d'un profil d'un patient. La prise en compte de ce champ permet de générer une probabilité de 0 pour certaines maladies exclues par la présence de ce signe.

L'invention permet également de prendre en considération un facteur de risque lié à la présence d'un antécédent, plus généralement d'un facteur $F_1$, $F_2$ ou $F_3$, qui s'applique à au moins une maladie ou un groupe de maladies. Les facteurs de risques pondèrent directement les probabilités conditionnelles associées aux maladies.

Un intérêt de considérer des seconds facteurs $F_2$, correspondant à des antécédents, est de prendre en considération des évènements passés d'un patient donné $P_1$ afin de calculer une probabilité conditionnelle d'une maladie donnée à l'existence de cet antécédent.

Selon un autre mode de réalisation qui se combine avec ce dernier, un antécédent est traité conjointement avec la prise en compte d'autres seconds facteurs $F_2$, c'est-à-dire d'autres antécédents lorsqu'ils existent. Selon un mode de réalisation, au moins un second facteur $F_2$ est corrélé à au moins un troisième facteur $F_3$, tel qu'un signe afin de calculer une probabilité conditionnelle prenant en considération différents types de facteurs $F_2$, $F_3$.

À cette fin le modèle bayésien permet de définir des liens logiques quantifiés entre les différents facteurs $F_1$, $F_2$, $F_3$ et les maladies permettant de pondérer les valeurs de probabilités conditionnelles de maladies Dans la définition de cette modélisation Bayésienne, l'invention permet de traiter les interactions entre différents types de facteurs $F_1$, $F_2$, $F_3$ de la même manière que si les facteurs étaient du même type. En effet, le modèle Bayésien de l'invention permet de modéliser les liens logiques sous forme de probabilités conditionnelles d'un événement à la survenance de facteurs, $F_1$, $F_2$, et/ou $F_3$.

On définit la probabilité $P(M \mid F_1, F_2)$ correspondant à la probabilité d'avoir la maladie M sachant la présence des facteurs $F_1$ et $F_2$.

On définit la probabilité $P(F_1, F_2 \mid M)$ correspondant à la probabilité d'avoir les facteurs $F_1$ et $F_2$ sachant la présence de la maladie M.

On définit la probabilité $P(F_1, F_2)$ correspondant à la probabilité d'avoir les facteurs $F_1$ et $F_2$.

La relation suivante est toujours vérifiée :

$$P(M \mid F_1, F_2) = P(F_1, F_2 \mid M) * P(M) / P(F_1, F_2)$$

La probabilité de ne pas avoir la maladie est notée $P(\text{ø}M)$ et la probabilité de ne pas avoir la maladie la maladie connaissant $F_1$ te $F_2$ est notée $P(\text{o}M \mid F_1, F_2)$. On a les relations suivantes : $P(\text{ø}M) = 1 - P(M)$ et $P(\text{ø}M \mid F_1, F_2) = 1 - P(M \mid F_1, F_2)$.

Selon un mode de réalisation de l'invention, les facteurs $F_1$, $F_2$ sont considérés comme indépendant. On obtient alors la relation suivante :

$$P(M \mid F_1, F_2) = P(F_1 \mid M) * P(F_2 \mid M) * P(M) / (P(F_1) * P(F_2))$$

$P(M)$ correspond à la probabilité d'avoir la maladie. Le procédé et/ou le système de l'invention déterminent comme valeur initiale la prévalence $PR_1$ ou l'incidence $IND_0$ de la maladie.

Lors de la détermination de la valeur initiale de $P(M)$, la prévalence $PR_1$ ou l'incidence $IND_1$ peuvent prendre en compte un certain nombre de facteurs de risque ou des données propres au profil du patient. Le procédé et le système de l'invention permettent de comparer ces informations aux données de la base des signes et/ou des maladies. Le procédé et le système de l'invention permettent alors de déterminer la valeur la plus pertinente de la prévalence ou de l'incidence afin de calculer la probabilité $P(M)$. À titre d'exemple si un patient de 60 ans est fumeur, la prévalence $PR_1$ qui sera déterminée prendra en considération la probabilité $P(M = \text{cancer du poumon})$ d'avoir le cancer du poumon pour un profil donné avec des antécédents donnés et des facteurs de risque donnés.

Selon un mode de réalisation, lorsque la prévalence $PR_1$ est définie dans la base de données d'une maladie M, la valeur est déterminée comme la valeur de $P(M)$. Lorsque la valeur n'est pas définie, la valeur de l'incidence $IN_1$ est choisie pour déterminer la valeur initiale de $P(M)$.

Selon un mode de réalisation, selon la maladie et le profil du patient {AGE, GEN, GEO, etc.} une règle de priorité est définie entre la prévalence et l'incidence. Cette règle permet de calculer $P(M)$, c'est-à-dire la probabilité d'avoir la maladie avec l'information la plus pertinente qui soit. Typiquement, dans un cas où la prévalence augmente avec l'âge, l'incidence peut paraitre définir une probabilité $P(M)$ plus pertinente que la prévalence pour certaines gammes d'âges du patient.

Les valeurs de spécificité et de sensibilité d'un ensemble de facteurs correspondant aux signes sont extraites d'une base de données pour calculer la probabilité d'un évènement donné, à savoir la présence d'une maladie M. Ces dernières valeurs quantifient les probabilités de troisièmes facteurs $F_3$ qui sont définies comme ci-après.

Lorsque la spécificité d'un ensemble de facteurs liés $F_1$, $F_2$ et $F_3$ pour une maladie est connue et enregistrée dans une base de données, alors la probabilité $P^*(M=1 \mid F_1, F_2, F_3)$ est directement sélectionnée dans la base quand les 3 facteurs sont acquis selon le procédé et/ou le système de l'invention. Si cette spécificité conjointe de ces facteurs n'est pas définie, le système et le procédé de l'invention permettent de calculer automatiquement la probabilité d'après la formule précédente en considérant les facteurs comme indépendants.

**Troisièmes facteurs $F_3$ : les signes**

Selon un mode de réalisation, l'interface $INT_1$ ou une autre interface permet l'acquisition $ACQ_3$ de troisièmes facteurs $F_3$. Les troisièmes facteurs $F_3$ comprennent la définition des signes $S_k$ génériques ou de signes détaillés $S_{ki}$. Lors de l'acquisition de données d'un nouveau signe $S_1$, l'interface $INT_1$, par exemple, permet d'extraire un signe de la base de signes BDs. Le signe $S_1$ est alors appelé par son concept ontologique le définissant, c'est-à-dire sa désignation, comme la « toux » ou la « fièvre ». Il s'agit du signe générique. Un intérêt est d'homogénéiser les signes sous un même concept. Le signe générique $S_k$ ou le signe détaillé $S_{ki}$ est alors renseigné par une pluralité de champs permettant de le préciser.

À titre d'exemple, la caractérisation de la présence d'un signe peut être précisée par différents paramètres. Selon un exemple, la fréquence d'apparition d'un signe peut être prise en compte et caractérisée. Cette caractérisation peut être qualifiée par un champ à déterminer parmi une liste prédéfinie de termes tels que : {ponctuel, sporadique, régulier, fréquent, continuellement}. La fréquence d'apparition d'un signe peut être conjointement ou alternativement renseignée par une courbe d'évolution. Un exemple de modification de l'apparition d'un signe peut être la suivante : apparition pendant 2 à 3 jours puis disparition pendant 6 à 10 jours et réapparition pendant 1 à 3 jours.

Sa fréquence, son intensité, sa localisation anatomique éventuelle visible ou « profonde » et décrite ou encore une qualification, etc., peuvent être prises en compte.

Les troisièmes facteurs peuvent donc être définis pour chaque patient en quantifiant un certain nombre de champs permettant de décrire le signe.

La figure 2 représente un exemple dans lequel un signe $S_k$ forme un concept commun à différentes manifestations de ce dernier, par exemple : une « toux sèche » ou une « toux grasse » ont pour concept commun la toux. L'ensemble des caractéristiques communes à tous les signes détaillés $S_{ki}$ forme les caractéristiques du signe générique $S_k$.

Dans l'exemple, de la figure 2, trois manifestations différentes $S_{K1}$, $S_{K2}$, $S_{K3}$ du signe $S_K$ sont représentées. Ces manifestations peuvent concerner un ou plusieurs éléments permettant de définir ou de spécifier le signe $S_k$. Dans la figure 2, une manifestation $S_{k1}$ du signe $S_k$ peut être plus ou moins enrichie : $S_{k1}(D_{k1}")$ représente une forme plus enrichie du signe $S_{k1}(D_{k1}')$ qui, lui-même, représente une forme enrichie du signe $S_{k1}(D_{k1})$. $D_{k1}$ représente ici la description du signe $S_{K1}$. Il peut s'agir par exemple d'une description plus précise d'une évolution du signe. L'invention permet de prendre en compte une valeur de spécificité d'un signe détaillé $S_{ki}$. Par défaut, lorsqu'elle n'est pas renseignée, la valeur est identique à la valeur du signe de plus haut niveau dans l'ontologie des signes. Dans le cas de la figure 2, la spécificité du signe $S_{k1}(D_{k1}")$ est égale à la spécificité du signe $S_{k1}(D_{k1}')$ si aucune valeur n'est associée à ce dernier lors de sa création.

Selon un mode de réalisation, la valeur de la spécificité peut être recalculée à partir d'une valeur de prévalence des maladies et de la sensibilité de l'ensemble des facteurs.

Dans la figure 2, la manifestation du signe $S_{k3}(D_{k3}')$ peut donner une description plus précise du signe $S_{k3}(D_{k3})$.

Un avantage de décrire au mieux un signe $S_1$ est d'augmenter sa spécificité $SP_1$ pour une maladie. L'invention permet de prendre en compte des descriptions enrichies afin de constituer une base de maladies la plus fiable possible. Ainsi, le procédé de l'invention permet de prendre en compte la construction d'une base enrichie de données de signes BDS.

Selon un mode de réalisation, le procédé de l'invention détermine à partir de la sensibilité $SE_1$ et de la spécificité $SP_1$ d'un signe, une liste de probabilités conditionnelles, chacune étant associée à une maladie. La prise en compte de différents antécédents et de différents signes et du profil du patient $P_1$ permet de modifier les probabilités $P(Mi)$ conditionnelles associées à chaque maladie $M_i$ de la liste qui est générée.

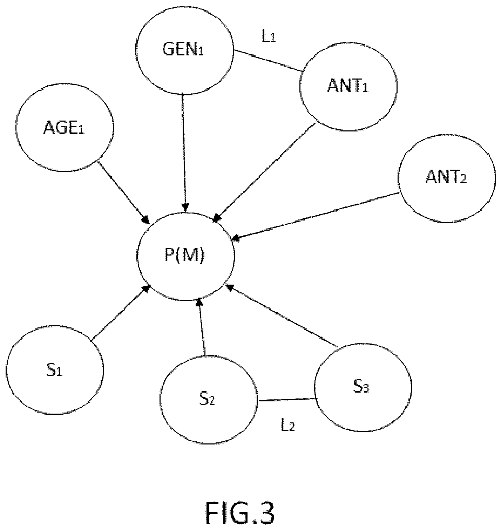

La figure 3 représente un exemple de modélisation du réseau bayésien permettant de calculer la probabilité $P(M)$ de survenue d'une maladie en fonction de la présence des premiers facteurs $F_1$ {$GEN_1$, $AGE_1$}, des seconds facteurs $F_2$ {$ANT_1$, $ANT_2$} et des troisièmes facteurs $F_3$ {$S_1$, $S_2$, $S_3$}.

Dans ce mode de réalisation, la représentation du réseau modélise un premier lien $L_1$ entre les facteurs $ANT_1$ et $GEN_1$ et un second lien $L_2$ entre les facteurs $S_2$ et $S_3$.

Selon un mode de réalisation, les liens correspondent à une survenue concomitante des facteurs lors de la présence de la maladie.

Les facteurs $S_2$ et $S_3$ sont liés et permettent de définir la probabilité conditionnelle suivante :
Selon un mode de réalisation, lorsque les facteurs $GEN_1$ et $ANT_1$ sont liés, le modèle de réseau bayésien permet de considérer la probabilité conditionnelle conjointe suivante : $P^*(M \mid F_1, F_2)$ directement à partir des valeurs de sensibilité et de spécificité de l'observation conjointe. Lorsque les facteurs ne sont pas liés, la probabilité de la maladie est alors calculée à partir des facteurs considérés comme indépendants.

On note alors $P^*(M \mid F_1, F_2)$ : la probabilité conjointe définie directement dans la base de données et $P(M \mid F_1, F_2)$ : la probabilité calculée en considérant que les facteurs $F_1$ et $F_2$ sont indépendants.

Un des avantages de l'invention est de permettre une modélisation comportant les valeurs des spécificités et de sensibilité conjointes de facteurs pouvant être liés. L'invention priorise alors dans les calculs les valeurs conjointes qui sont définies dans la base de données lorsqu'elles sont connues.

Le système et le procédé de l'invention permettent de prendre en compte une modélisation d'un réseau bayésien naïf augmenté dans lequel un contrôle de présence de certaines valeurs de probabilités est effectué lorsque des facteurs sont susceptibles d'être liés. À titre d'exemple, si un facteur $F_1$ est acquis par une saisie utilisateur et que ce facteur est lié à un second facteur $F_2$, par exemple par la présence dans la base d'une probabilité conjointe liée à une maladie définie, alors le système et le procédé de l'invention permettent de contrôler la présence du second facteur $F_2$ dans les champs saisis par un utilisateur ou de générer automatiquement une interface afin d'obtenir de l'utilisateur une information qualifiant la présence ou non d'un second facteur $F_2$.

Dans ce dernier mode de réalisation, les facteurs liés peuvent l'être en étant dans l'un des trois ensembles $ENS_1$, $ENS_2$, $ENS_3$.

La probabilité d'avoir une maladie M est alors déduite de l'ensemble des données décrivant les premiers, seconds et troisièmes facteurs supposés être observés ou étant observés. Selon ce mode de réalisation, les probabilités des facteurs sont alors déduites des descriptions et des valeurs les décrivant ou les définissant. Les valeurs de spécificité et de sensibilité servent à calculer la table des probabilités selon les facteurs considérés.

Pour un facteur donné, $F_1$, on considère dans cet exemple, une valeur de la sensibilité Se de 5 et une valeur de la spécificité Sp de 4 sur une échelle de 0 à 6, soit en valeur réelle après conversion Se=0,875 et Sp = 0,7.

Selon cet exemple, nous avons donc la table de probabilité suivante :

|  | $F_1 = 0$ | $F_1 = 1$ |
|---|---|---|
| M = 0 | $P(M=0 \mid F_1=0) = (1+ Sp_1) / 2 = 0,85$ | $P(M=0 \mid F_1=1) = (1 - Sp_1) / 2 = 0,15$ |
| M = 1 | $P(M=1 \mid F_1=0) = 1 - Se_1 = 0,125$ | $P(M=1 \mid F_1=1) = Se_1 = 0,875$ |

Avec :

- M = 0 : absence de maladie M ;
- M = 1 : présence de la maladie M ;
- $F_1 = 0$ : absence du facteur $F_1$ ;
- $F_1 = 1$ : présence du facteur $F_1$.

**Exemple de la maladie de Horton**

L'invention permet de décrire une maladie en la désignant et en lui associant un descriptif. Dans le cas de la maladie de Horton, le descriptif peut indiquer un nom clinique ou un nom d'usage de la maladie tel que : la maladie est connue aussi sous le nom d'« artérite temporale ». Une courte description comme le type de maladie : « maladie inflammatoire des vaisseaux » peut être indiquée dans la base de données des maladies BDM. Une information quantifiant la prévalence et l'incidence peut, en outre, être indiquée comme : la maladie de Horton touche particulièrement les sujets âgés. Elle est connue aussi sous le nom d'« artérite temporale », du fait que l'une de ces artères (temporale superficielle droite ou gauche) est affectée au cours de la maladie.

Les informations décrivant la maladie peuvent comprendre en outre :

- un degré d'urgence ;
- une forme clinique du tableau principal ;
- une prévalence associée à au moins une population comportant une démographie, ladite population étant segmentée selon un premier modèle ;
- une incidence associée à au moins une population comportant une démographie, ladite population étant segmentée selon un premier modèle ;
- une description ;
- une distribution démographique associée à des facteurs de risques.

Selon un mode de réalisation de l'invention, dans une modélisation du réseau bayésien, les facteurs sont considérés comme étant indépendants.

Dans cet exemple on considère un le diagnostic suivant :

Diagnostic de la Maladie1 : la Maladie de Horton

La prévalence $PR_1$ de la maladie est de 1 sur 11 000 dans l'ensemble de la population.

La prévalence $PR_2$ est de 10/10 000 au-delà de 60 ans. La prévalence $PR_2$ est sélectionnée automatiquement dans la base lorsque l'âge de l'individu est supérieur à 60 ans. Dans ce cas de figure, le système et/ou le procédé de l'invention déterminent automatiquement la valeur enregistrée dans la base de données de facteurs liés. Ici l'âge est considéré comme un facteur de risque augmentant la probabilité d'avoir la maladie. Ce facteur est lié à la prévalence initiale $PR_1$ ou $P(M)$.

La maladie est répartie avec un rapport Femme/Homme de 2/3 - 1/3.

Un patient $P_1$ manifeste les signes suivants (**Facteurs $F_3$**) :

- **Signe 1** = Céphalées matinales
- **Signe 2** = Fièvre isolée : Spécificité (Sp1) : <5% pour la maladie de Horton / Sensibilité (Se1): 90% dans la maladie de Horton ;
- **Signe 3** = Fatigue isolée: Spécificité (Sp2) : <5% pour la maladie de Horton / Sensibilité (Se2): 60% dans la maladie de Horton ;
- **Signe 4** = Amaigrissement sans autre cause : Spécificité (Sp3) : 15% pour la maladie de Horton / Sensibilité (Se3): 50% dans la maladie de Horton ;
- **Signe 5** = Douleur à la palpation de l'artère temporale (du côté de la céphalée) : Spécificité (Sp4) : 75% pour la maladie de Horton / Sensibilité (Se4) : 40% dans la maladie de Horton.

On considère le profil du Patient $P_1$ dont les facteurs $F_1$ comprennent l'âge $AGE_1$ = 72 ans, le genre $GEN_1$ = femme et une information géographique $GEO_1$ = Paris, France. Les seconds facteurs $F_2$ comprennent un antécédent $ANT_1$.

Le procédé génère une liste de maladies avec des probabilités associées, Liste 1 :

Maladie de Horton : Proba1 %
Maladie 2 : Proba2 %
Maladie 3 : Proba3 %
Maladie 4 : Proba4 %
Maladie 5 : Proba5 %

La probabilité $P(M)$ avec M étant la maladie de Horton peut s'écrire comme une fonction « f » de différents paramètres et de variables :

$$P(M \mid \{Fi\}i\epsilon[1;N]) = f(ANT_1, Sp_1, Sp_2, Sp_3, Sp_4, Se_1, Se_2, Se_3, Se_4,$$

$(PR_1$ ou $PR_2$ ou $IN_1))$

On définit $P(S=0 \mid M=0)$ la probabilité que l'évènement « S » ne se produise pas sachant que la maladie n'est pas présente.

$$P(S=0 \mid M=0) = (1+ Sp_1) / 2$$

$$P(S=1 \mid M = 0) = (1 - Sp_1) / 2$$

$$P(S=1 \mid M= 1) = Se$$

$$P(S=0 \mid M=1) = 1 - Se_1$$

Pour chaque signe Si et antécédent $ANT_i$, on calcule la table de ces 4 probabilités qui servent à alimenter chaque noeud du modèle ; la distribution conjointe est calculée à partir de ces tables de probabilité.

Ces quatre relations sont donc écrites pour chaque valeur de spécificité $Sp_i$ et de sensibilité $Se_i$ de chaque signe Si.

## Prise en Compte des maladies rares

Selon un mode de réalisation, l'interface du système de l'invention permet de configurer le nombre de maladies associées à une probabilité qui est affiché dans une liste $LIST_1$. Par défaut, cette valeur peut être définie à 5.

Selon un mode de réalisation, un onglet permettant d'activer ou de désactiver la prise en compte de maladies rares est présent sur l'interface. Ainsi, un utilisateur peut décider d'afficher des maladies associées à une faible probabilité du fait de leur rareté. Cette option permet notamment de vérifier qu'un ensemble de signes et le profil d'un patient peuvent être associés à une maladie rare.

Un autre avantage est de permettre à un utilisateur de conduire une action adaptée si des indices renforcent la possibilité d'une maladie ayant une faible probabilité chez le patient. Par exemple, une action adaptée pourrait être de recommander des tests complémentaires au patient afin d'écarter la présence d'une maladie rare.

## Interface d'accès

Selon un mode de réalisation, la liste $LIST_1$ de maladies qui est générée par le système ou le procédé de l'invention génère un ensemble d'icônes permettant d'accéder à une fiche associée à la maladie sélectionnée dans la liste $LIST_1$. La fiche permet de présenter à un utilisateur l'ensemble des caractéristiques de la maladie qui est renseignée dans la base.

En particulier, la fiche permet d'afficher les valeurs de spécificité et de sensibilité des signes présents dans la maladie.

Ainsi, l'utilisateur peut analyser les données de chaque signe individuellement.

## Pharmacovigilance

Selon un exemple de réalisation, le procédé de l'invention permet de prendre en compte des quatrièmes facteurs $F_4$ dans le calcul des probabilités conditionnelles associées aux maladies générées dans une liste $LIST_1$. Les quatrièmes facteurs $F_4$ sont définis lors d'une quatrième acquisition d'information $ACQ_4$. La quatrième acquisition $ACQ_4$ d'informations comprend le nom d'un principe actif et au moins la présence ou non d'un effet connu.

Le système de l'invention comprend une base de données de principes actifs qui peuvent être associés ou non à des produits tels que des médicaments. Une mémoire permet d'enregistrer la probabilité qu'un signe soit lié à la prise d'un médicament ou qu'une maladie soit liée à la prise d'un médicament. La probabilité est alors prédéfinie pour une population donnée.

L'interface du système de l'invention permet de prendre en compte la prise de médicaments par au moins un patient. Selon un mode de réalisation, la durée et les dates du traitement et la posologie peuvent être renseignées dans l'interface. Les données sont alors enregistrées et associées à un patient donné.

Le calcul des probabilités des maladies de la liste $LIST_1$ est alors pondéré par les probabilités qu'un signe, un groupement de signes ou une maladie soit lié(e) à la prise d'un médicament selon les propriétés renseignées à savoir : nom du produit, durée et dates du traitement, posologie, etc.

Selon un autre aspect, l'invention permet de prendre en compte les effets résultants de la prise d'un principe actif, tel qu'un médicament, pour en déduire les relations causales avec la survenue d'effets ou non chez un ou des patients.

Ce mode de réalisation permet de dissocier les effets provenant de la prise d'un premier principe actif qui produit au moins un effet pharmacologique identifié d'un second principe actif ou d'un placebo qui ne produit pas cet effet pharmacologique.

La figure 1 introduit l'acquisition des informations de la quatrième acquisition $ACQ_4$ comme un évènement $EVN_1$.

Un intérêt de ce mode de réalisation est de réaliser des tests sur deux ensembles de patients, $ENS_{MED}$ et $ENS_{PBO}$, dont un ensemble $ENS_{MED}$ comprend les patients ayant reçu un médicament et un ensemble $ENS_{PBO}$ comprend les patients ayant reçu le placebo. Les tests visent à quantifier les incidences des signes, affections émergentes et indésirables, validés par des médecins investigateurs de recherches cliniques pour chacun des deux ensembles, d'en évaluer les différences d'incidences statistiquement significatives et d'en évaluer l'imputabilité éventuelle à un médicament sur l'apparition d'un signe $S_k$ ou d'une maladie $M_i$ chez un patient en général.

Le procédé est alors répété une pluralité de fois pour chaque patient des deux ensembles. Les valeurs peuvent être par exemple moyennées sur l'ensemble des patients d'un même ensemble. Selon un autre mode de réalisation, d'autres fonctions peuvent être utilisées telles qu'une fonction médiane. Les probabilités conditionnelles moyennées de chaque maladie de chaque agrégation de liste selon les ensembles considérés sont alors comparées. Le procédé de l'invention permet alors de générer automatiquement au moins un indicateur traçant un écart entre deux probabilités moyennées associées à une même maladie dans chaque liste agrégée de chaque groupe. Un avantage est de mesurer les écarts significatifs permettant d'isoler des effets liés à la prise d'un médicament pour un groupe de patients étant donné qu'il ne produit pas le même effet chez les patients de l'ensemble $ENS_{PBO}$.

Selon un mode de réalisation, la quatrième acquisition de données $ACQ_4$ comporte une description d'un ensemble de signes $S_k$ associés à la prise d'un médicament. À ce titre, les données de ces signes $S_k$ font partie de l'ensemble de données $ENS_{MED}$. Les signes $S_k$ sont alors pris en compte dans le calcul des probabilités conditionnelles de la liste $LIST_1$. Ici, les valeurs de sensibilité $SE_k$ et de spécificité $SP_k$ relativement à un ensemble de maladies sont intégrées dans le modèle. Un intérêt est de mesurer l'influence de la prise d'un médicament dans l'apparition d'une maladie. En d'autres termes, il est possible de diminuer les erreurs de diagnostic en isolant les effets issus de la prise de médicament.

Selon un mode de réalisation, l'invention génère :

- une première liste $LIST_1$ comportant les probabilités conditionnelles prenant en compte les signes associés au médicament administré par un patient $P_1$ et ;
- une seconde liste $LIST_2$ comportant les probabilités conditionnelles ne prenant pas en compte les signes associés au médicament pris par un patient $P_1$.

Un utilisateur peut alors déduire directement, s'il existe, l'effet d'un médicament dans l'apparition d'une maladie identifiée

## Revendications

1. Système (1) comportant un calculateur pour la génération d'une liste de probabilités ($LIST_1$) associée à une liste de maladies pour un premier patient ($P_1$), ledit système comportant une interface permettant :

   - Une première acquisition ($ACQ_1$) d'un premier ensemble ($ENS_1$) de premiers facteurs ($F_1$) relatif au premier patient ($P_1$) et enregistrement desdits premiers facteurs ($F_1$) dans une mémoire, lesdits premiers facteurs ($F_1$) comportant :

      ◦ une valeur d'âge ($AGE_1$),
      ◦ une valeur de genre ($GEN_1$) ;

   - une seconde acquisition ($ACQ_2$) d'un second ensemble ($ENS_2$) de seconds facteurs ($F_2$), décrivant notamment au moins une maladie ($ANT_1$) ou une information propre audit patient ($P_1$) et un enregistrement desdits seconds facteurs ($F_2$) dans une mémoire, ladite maladie ($ANT_1$) étant extraite d'une première base de données ($BD_M$), chaque maladie ($ANT_1$) étant associée à une première statistique de prévalence ($PR_1$) et/ou d'une première statistique d'incidence ($IN_1$), et chaque maladie étant associé à une liste de signes ($S_k$), au moins un signe correspondant à un symptôme d'une maladie ;
   - une troisième acquisition ($ACQ_3$) d'un troisième ensemble ($ENS_3$) de troisièmes facteurs ($F_3$) décrivant au moins un signe ($S_1$) et enregistrement desdits troisièmes facteurs ($F_3$) dans une mémoire du système, ledit premier signe ($S_1$) comportant une première statistique de sensibilité (SEi) et une seconde statistique de spécificité (SPi) pour chaque maladie d'une liste prédéfinie ($LIST_1$) de maladies (Mi) associée audit signe ($S_1$), la sensibilité étant modélisée selon une échelle de valeurs prédéfinies, chaque valeur prédéfinie correspondant à une valeur d'une statistique de sensibilité du signe pour une maladie donnée, la spécificité étant modélisée selon une échelle de valeurs prédéfinies, chaque valeur prédéfinie de l'échelle correspondant à une valeur d'une statistique de spécificité du signe pour une maladie donnée;
   ledit calculateur générant, à partir d'une première modélisation d'un réseau Bayésien (RB) et de données d'entrées comportant les données de la première, seconde et de la troisième acquisition ($ACQ_1$, $ACQ_2$, $ACQ_3$), un ensemble de probabilités, chaque probabilité étant associé à une maladie donnée de la première liste ($LIST_1$) ; ledit système (1) comportant une interface graphique pour afficher ladite première liste générée ($LIST_1$).

2. Système selon la revendication 1, **caractérisé en ce qu'**il comprend une interface permettant une quatrième acquisition ($ACQ_4$) de quatrièmes facteurs ($F_4$) décrivant un produit médical donné ($PROD_1$) et au moins un second signe ($S_2$) associé audit produit médical donné ($PROD_1$), ledit second signe ($S_2$) comportant une première statistique de sensibilité ($SE_i$,) et une seconde statistique de spécificité (SPi) pour chaque maladie d'une seconde liste prédéfinie ($LIST_2$) de maladies associée audit second signe ($S_2$) ; le calculateur générant l'ensemble de probabilités en prenant en compte en entrées les données de la quatrième acquisition ($ACQ_4$) pour générer la première liste ($LIST_1$).

3. Système (1) selon la revendication 2, **caractérisé en ce qu'**il comporte une mémoire pour enregistrer des données décrivant au moins deux produits médicaux (PROD$_1$, PROD$_2$) et enregistrer des données provenant d'une pluralité d'acquisitions de données provenant d'un ensemble de patients (P$_j$) de telle sorte qu'un premier groupe de patients (GR$_1$) soit associé au premier produit (PROD$_1$) et un second groupe de patients (GR$_2$) soit associé au second produit (PROD$_2$), le calculateur générant deux listes de probabilités conditionnelles, chaque probabilité étant associée à une maladie donnée, le calculateur effectuant un calcul pour comparer les deux listes afin de déduire la présence d'au moins un écart de probabilités pour une même maladie entre les deux listes lorsque ledit écart est supérieur à un seuil prédéfini.

4. Système (1) selon l'une quelconque des revendications 1 à 3, **caractérisé en ce qu'**il comprend une ressource linguistique comportant une ontologie, un dictionnaire ou une base de synonymes permettant d'associer des termes décrivant des signes ou des maladies dans la base de données (BDM, BDS) à un corpus de textuel prédéfini.

5. Système (1) selon l'une quelconque des revendications 1 à 4, **caractérisé en ce que** chaque troisième facteur (F$_3$) comporte une pluralité de propriétés décrivant un signe et enregistrées dans une mémoire du système, chaque propriété étant associée à une valeur de sensibilité et de spécificité.

6. Système (1) selon l'une quelconque des revendications 1 à 5, **caractérisé en ce que** la première modélisation du réseau Bayésien (RB) comporte, pour au moins un noeud du réseau, une modélisation d'au moins un lien entre deux facteurs d'un des quatre ensembles de facteurs (F$_1$, F$_2$, F$_3$, F$_4$), ladite modélisation spécifiant si les facteurs sont indépendants ou dépendants et étant enregistrée dans une mémoire du système.

7. Système (1) selon la revendication 6, **caractérisé en ce que** :

   ▪ lorsqu'aucun lien de dépendance entre au moins deux facteurs présents dans les données acquises (ACQ1, ACQ$_2$, ACQ$_3$ ACQ$_4$) n'est spécifié, le calcul de la probabilité d'une maladie comprend un calcul de probabilité conditionnelle (P(M| F$_i$, F$_k$) à partir des facteurs considérés comme indépendants ;
   ▪ lorsque ladite modélisation bayésienne spécifie un lien de dépendance entre au moins deux facteurs (F$_i$, F$_k$), la probabilité d'une maladie comprend une sélection dans la base de données soit de la de probabilité conditionnelle conjointe soit une sélection d'une valeur de sensibilité et/ou de spécificité des facteurs considérés conjointement.

8. Système (1) selon l'une quelconque des revendications 2 à 7, **caractérisé en ce qu'**il comprend un calculateur pour établir une comparaison entre les données acquises (ACQ1, ACQ$_2$, ACQ$_3$ ACQ$_4$) par l'intermédiaire de l'interface et les données enregistrées dans la mémoire de sorte que lorsqu'un facteur de risque (F$_2$) acquis est associé à une statistique de prévalence (PR) ou d'incidence (IN) d'une maladie (M), la probabilité de la maladie associée (P(M)) est initialisée à la valeur enregistrée dans la base de données.

9. Procédé de génération d'une liste de probabilités (LIST$_1$) associée à une liste de maladies pour un premier patient (P$_1$), ledit procédé comportant l'utilisation d'une interface pour :

   ▪ Une première acquisition (ACQ$_1$) d'un premier ensemble de premiers facteurs (F$_1$) relatif au premier patient (P$_1$) comportant :

      ◦ une valeur d'âge (AGE$_1$),
      ◦ une valeur de genre (GEN$_1$) ;

   ▪ une seconde acquisition (ACQ$_2$) d'un second ensemble (ENS$_2$) de seconds facteurs (F$_2$), dits facteurs de risques, décrivant notamment au moins une maladie (ANT$_1$) ou une information propre audit patient (P$_1$) dudit patient (P$_1$), ladite maladie (ANT$_1$) étant extraite d'une première base de données (BD$_M$), chaque maladie (ANT$_1$) étant associée à une première statistique de prévalence (PR$_1$) et d'une première statistique d'incidence (IN$_1$), et chaque maladie étant associée à une liste de signes ;
   ▪ une troisième acquisition (ACQ$_3$) d'un troisième ensemble (ENS$_3$) de troisièmes facteurs (F$_3$) décrivant au moins un signe (S$_1$), ledit premier signe (S$_1$) comportant une première statistique de sensibilité (SE$_i$,) et une seconde statistique de spécificité (SPi) pour chaque maladie d'une liste prédéfinie (LIST$_1$) de maladies (Mi) associée audit signe (S$_1$) ;

ledit procédé comprenant en outre l'utilisation d'un calculateur pour l'application d'une première modélisation d'un réseau Bayésien (RB) à des données d'entrées comportant les données de la première, seconde et de la troisième

acquisition (ACQ$_1$, ACQ$_2$, ACQ$_3$), pour la génération d'un ensemble de probabilités, chaque probabilité étant associé à une maladie donnée de la première liste (LIST$_1$).

10. Procédé selon la revendication 9, **caractérisé en ce que** la probabilité associée à une maladie de la première liste (LIST$_1$) est une probabilité conditionnelle d'une maladie à la survenance d'un ensemble de facteurs (F$_1$, F$_2$, F$_3$) comportant au moins trois éléments parmi la liste suivante :

- un signe prédéfini (S$_k$) ;
- un antécédent prédéfini (ANT$_k$) ;
- un facteur de risque (F$_2$) ;
- un âge prédéfini (AGE$_k$) ;
- un genre prédéfini (GEN$_k$) ;
- un lieu géographique prédéfini (GEO$_k$) ;

11. Procédé selon l'une quelconque des revendications 9 à 10, **caractérisé en ce qu'**au moins un facteur (F$_2$) de la seconde acquisition (ACQ$_2$) de données est associé à un antécédent (ANT1) et comporte au moins un des critères suivants :

- une première date d'apparition et/ou ;
- une fréquence d'apparition et/ou ;
- une information génétique.

12. Procédé selon l'une quelconque des revendications 9 à 11, **caractérisé en ce que** la première modélisation du réseau Bayésien (RB) comporte, pour au moins un noeud du réseau, une modélisation d'au moins un lien entre deux facteurs d'un des quatre ensembles de facteurs (F$_1$, F$_2$, F$_3$, F$_4$), ladite modélisation (RB) spécifiant si les facteurs sont indépendants ou dépendants, la relation de dépendance entre au moins deux facteurs étant modélisée par une valeur de probabilité conditionnelle conjointe d'une maladie sachant les facteurs présents.

13. Procédé selon l'une quelconque des revendications 9 à 12, **caractérisé en ce qu'**il comprend une étape de vérification de l'existence d'un lien entre facteurs acquis des différents ensembles (ENS$_1$, ENS$_2$, ENS$_3$) dans la base de données des signes ou des maladies, le cas échéant, le procédé comprend une étape de sélection de la valeur de la probabilité conjointe associée aux facteurs liés.

14. Procédé selon l'une quelconque des revendications 9 à 13, **caractérisé en ce qu'**il comprend une quatrième acquisition (ACQ$_4$) de quatrièmes facteurs (F$_4$) décrivant un produit médical donné (PROD$_1$) et au moins un second signe (S$_2$) associé audit produit médical donné (PROD$_1$), ledit second signe (S$_2$) comportant une première statistique de sensibilité (SE$_i$,) et une seconde statistique de spécificité (SPi) pour chaque maladie d'une seconde liste prédéfinie (LIST$_2$) de maladies associée audit second signe (S$_2$) ; l'étape de génération (GEN) de l'ensemble de probabilités prenant en compte en entrées les données de la quatrième acquisition (ACQ$_4$).

15. Produit programme d'ordinateur chargeable directement dans la mémoire interne d'un ordinateur numérique, comprenant des portions de code de logiciel pour l'exécution des étapes du procédé selon l'une quelconque des revendications 9 à 14 lorsque ledit programme est exécuté sur un ordinateur.

**Patentansprüche**

1. System (1), das einen Rechner zur Generierung einer Wahrscheinlichkeitsliste (LIST$_1$) aufweist, die einer Krankheitsliste für einen ersten Patienten (P$_1$) zugeordnet ist, wobei das System eine Schnittstelle aufweist, die gestattet:

- eine erste Erfassung (ACQ$_1$) einer ersten Gruppe (ENS$_1$) erster Faktoren (F$_1$) in Bezug auf den ersten Patienten (P$_1$) und Speicherung der ersten Faktoren (F$_1$) in einem Speicher, wobei die ersten Faktoren (F$_1$) aufweisen:

  ○ einen Alterswert (AGE$_1$),
  ○ einen geschlechtsspezifischen Wert (GEN$_1$);

- eine zweite Erfassung (ACQ$_2$) einer zweiten Gruppe (ENS$_2$) zweiter Faktoren (F$_2$), die insbesondere min-

destens eine Krankheit ($ANT_1$) oder eine für diesen Patienten spezifische Information ($P_1$) beschreiben und eine Speicherung der zweiten Faktoren ($F_2$) in einem Speicher, wobei die Krankheit ($ANT_1$) aus einer ersten Datenbank ($BD_M$) extrahiert ist, wobei jede Krankheit ($ANT_1$) einer ersten Prävalenzstatistik ($PR_1$) und/oder einer ersten Inzidenzstatistik ($IN_1$) zugeordnet ist, wobei jede Krankheit einer Liste von Anzeichen ($S_k$) zugeordnet ist, wobei mindestens ein Anzeichen einem Symptom einer Krankheit entspricht;

■ eine dritte Erfassung ($ACQ_3$) einer dritten Gruppe ($ENS_3$) dritter Faktoren ($F_3$), die mindestens ein Anzeichen ($S_1$) beschreiben und Speicherung der dritten Faktoren ($F_3$) in einem Speicher des Systems, wobei das erste Anzeichen ($S_1$) eine erste Sensitivitätsstatistik (SEi) und eine zweite Spezifitätsstatistik (SPi) für jede Krankheit einer vordefinierten Liste ($LIST_1$) von Krankheiten (Mi) aufweist, die dem Anzeichen ($S_1$) zugeordnet ist, wobei die Sensitivität gemäß einer Skala vordefinierter Werte modelliert ist, wobei jeder vordefinierte Wert einem Wert einer Sensitivitätsstatistik des Anzeichens für eine bestimmte Krankheit entspricht, wobei die Spezifität gemäß einer Skala vordefinierter Werte modelliert ist, wobei jeder vordefinierte Wert der Skala einem Wert einer Spezifitätsstatistik des Anzeichens für eine bestimmte Krankheit entspricht;

wobei der Rechner auf der Basis einer ersten Modellierung eines Bayesschen Netzes (RB) und Eingangsdaten, die die Daten der ersten, zweiten und dritten Erfassung ($ACQ_1$, $ACQ_2$, $ACQ_3$) aufweisen, eine Gruppe von Wahrscheinlichkeiten generiert, wobei jede Wahrscheinlichkeit einer bestimmten Krankheit der ersten Liste ($LIST_1$) zugeordnet ist;

wobei das System (1) eine grafische Schnittstelle zum Anzeigen der generierten ersten Liste ($LIST_1$) aufweist.

2. System nach Anspruch 1, **dadurch gekennzeichnet, dass** es eine Schnittstelle umfasst, die eine vierte Erfassung ($ACQ_4$) vierter Faktoren ($F_4$) gestattet, die ein bestimmtes Medizinprodukt ($PROD_1$) und mindestens ein zweites Anzeichen ($S_2$) beschreiben, das diesem bestimmten Medizinprodukt ($PROD_1$) zugeordnet ist, wobei das zweite Anzeichen ($S_2$) eine erste Sensitivitätsstatistik ($SE_{i,}$) und eine zweite Spezifitätsstatistik (SPi) für jede Krankheit einer zweiten vordefinierten Krankheitsliste ($LIST_2$) aufweist, die diesem zweiten Anzeichen ($S_2$) zugeordnet ist; wobei der Rechner die Gesamtheit der Wahrscheinlichkeiten unter Berücksichtigung der eingegebenen Daten der vierten Erfassung ($ACQ_4$) generiert, um die erste Liste ($LIST_1$) zu generieren.

3. System (1) nach Anspruch 2, **dadurch gekennzeichnet, dass** es einen Speicher zum Speichern der Daten aufweist, die mindestens zwei Medizinprodukte ($PROD_1$, $PROD_2$) beschreiben, und zum Speichern von Daten, die aus einer Vielzahl von Erfassungen von Daten stammen, die von einer Gruppe von Patienten ($P_j$) stammen, so dass eine erste Patientengruppe ($GR_1$) dem ersten Produkt ($PROD_1$) zugeordnet ist und eine zweite Patientengruppe ($GR_2$) dem zweiten Produkt ($PROD_2$) zugeordnet ist, wobei der Rechner zwei Listen bedingter Wahrscheinlichkeiten generiert, wobei jede Wahrscheinlichkeit einer bestimmten Krankheit zugeordnet ist, wobei der Rechner eine Berechnung durchführt, um die beiden Listen zu vergleichen, um das Vorhandensein von mindestens einer Wahrscheinlichkeitsabweichung für dieselbe Krankheit zwischen den beiden Listen abzuleiten, wenn die Abweichung einen vorgegebenen Schwellenwert überschreitet.

4. System (1) nach einem der Ansprüche 1 bis 3, **dadurch gekennzeichnet, dass** es eine Sprachressource umfasst, die eine Ontologie, ein Wörterbuch oder eine Synonymdatenbank aufweist, die es gestattet, Begriffe, die Anzeichen oder Krankheiten in der Datenbank (BDM, BDS) beschreiben, einem vordefinierten Textkörper zuzuordnen.

5. System (1) nach einem der Ansprüche 1 bis 4, **dadurch gekennzeichnet, dass** jeder dritte Faktor ($F_3$) eine Vielzahl von Eigenschaften aufweist, die ein Anzeichen beschreiben und in einem Speicher des Systems gespeichert sind, wobei jede Eigenschaft einem Sensitivitäts- und Spezifitätswert zugeordnet ist.

6. System (1) nach einem der Ansprüche 1 bis 5, **dadurch gekennzeichnet, dass** die erste Modellierung des Bayesschen Netzes (RB) für mindestens einen Knoten des Netzes eine Modellierung von mindestens einer Verbindung zwischen zwei Faktoren einer der vier Faktorengruppen ($F_1$, $F_2$, $F_3$, $F_4$) aufweist, wobei die Modellierung spezifiziert, ob die Faktoren unabhängig oder abhängig sind und in einem Speicher des Systems gespeichert ist.

7. System (1) nach Anspruch 6, **dadurch gekennzeichnet, dass**:

■ wenn kein Abhängigkeitsverhältnis zwischen mindestens zwei Faktoren, die in den erfassten Daten ($ACQ_1$, $ACQ_2$, $ACQ_3$, $ACQ_4$) vorhanden sind, spezifiziert ist, die Berechnung der Wahrscheinlichkeit einer Krankheit eine Berechnung einer bedingten Wahrscheinlichkeit ($P(M|F_i, F_k)$ auf der Basis der als unabhängig betrachteten Faktoren umfasst;

■ wenn die Bayessche Modellierung ein Abhängigkeitsverhältnis zwischen mindestens zwei Faktoren ($F_i$, $F_k$) spezifiziert, die Wahrscheinlichkeit einer Krankheit eine Auswahl in der Datenbank entweder der gemeinsamen bedingten Wahrscheinlichkeit oder eine Auswahl eines Sensitivitäts- und/oder Spezifitätswerts der gemeinsam betrachteten Faktoren umfasst.

8. System (1) nach einem der Ansprüche 2 bis 7, **dadurch gekennzeichnet, dass** es einen Rechner umfasst, um einen Vergleich zwischen den über die Schnittstelle erfassten Daten ($ACQ_1$, $ACQ_2$, $ACQ_3$, $ACQ_4$) und den im Speicher gespeicherten Daten herzustellen, so dass, wenn ein erfasster Risikofaktor ($F_2$) einer Prävalenzstatistik (PR) oder Inzidenzstatistik (IN) einer Krankheit (M) zugeordnet ist, die Wahrscheinlichkeit der zugeordneten Krankheit (P(M)) auf den in der Datenbank gespeicherten Wert initialisiert wird.

9. Verfahren zur Generierung einer Wahrscheinlichkeitsliste ($LIST_1$), die einer Krankheitsliste für einen ersten Patienten ($P_1$) zugeordnet ist, wobei das Verfahren die Verwendung einer Schnittstelle umfasst für:

- eine erste Erfassung ($ACQ_1$) einer ersten Gruppe erster Faktoren ($F_1$) in Bezug auf den ersten Patienten ($P_1$), die aufweist:

  ◦ einen Alterswert ($AGE_1$),
  ◦ einen geschlechtsspezifischen Wert ($GEN_1$);

- eine zweite Erfassung ($ACQ_2$) einer zweiten Gruppe ($ENS_2$) zweiter Faktoren ($F_2$), bezeichnet als Risikofaktoren, die insbesondere mindestens eine Krankheit ($ANT_1$) oder eine für diesen Patienten ($P_1$) spezifische Information des Patienten ($P_1$) beschreiben, wobei die Krankheit ($ANT_1$) aus einer ersten Datenbank ($BD_M$) extrahiert ist, wobei jede Krankheit ($ANT_1$) einer ersten Prävalenzstatistik ($PR_1$) und einer ersten Inzidenzstatistik ($IN_1$) zugeordnet ist, und wobei jede Krankheit einer Anzeichenliste zugeordnet ist;
- eine dritte Erfassung ($ACQ_3$) einer dritten Gruppe ($ENS_3$) dritter Faktoren ($F_3$), die mindestens ein Anzeichen ($S_1$) beschreiben, wobei das erste Anzeichen ($S_1$) eine erste Sensitivitätsstatistik ($SE_i$,) und eine zweite Spezifitätsstatistik (SPi) für jede Krankheit einer vordefinierten Liste ($LIST_1$) von Krankheiten ($M_i$) aufweist, die diesem Anzeichen ($S_1$) zugeordnet ist;

wobei das Verfahren ferner die Verwendung eines Rechners für die Anwendung einer ersten Modellierung eines Bayesschen Netzes (RB) auf Eingangsdaten, die die Daten der ersten, zweiten und der dritten Erfassung ($ACQ_1$, $ACQ_2$, $ACQ_3$) enthalten, für die Generierung einer Gruppe von Wahrscheinlichkeiten umfasst, wobei jede Wahrscheinlichkeit einer bestimmten Krankheit der ersten Liste ($LIST_1$) zugeordnet ist.

10. Verfahren nach Anspruch 9, **dadurch gekennzeichnet, dass** die einer Krankheit der ersten Liste ($LIST_1$) zugeordnete Wahrscheinlichkeit eine bedingte Wahrscheinlichkeit einer Krankheit beim Auftreten einer Gruppe von Faktoren ($F_1$, $F_2$, $F_3$) mit mindestens drei Elementen aus der folgenden Liste ist:

- ein vordefiniertes Anzeichen ($S_k$);
- eine vordefinierte Vorgeschichte ($ANT_k$);
- ein Risikofaktor ($F_2$);
- ein vorgegebenes Alter ($AGE_k$);
- eine vordefiniertes Geschlechtsspezifik ($GEN_k$);
- ein vordefinierter geografischer Standort ($GEO_k$).

11. Verfahren nach einem der Ansprüche 9 bis 10, **dadurch gekennzeichnet, dass** mindestens ein Faktor ($F_2$) der zweiten Datenerfassung ($ACQ_2$) einer Vorgeschichte (ANT1) zugeordnet ist und mindestens eines der folgenden Kriterien umfasst:

- ein Datum des ersten Auftretens; und/oder
- eine Häufigkeit des Auftretens; und/oder
- genetische Informationen.

12. Verfahren nach einem der Ansprüche 9 bis 11, **dadurch gekennzeichnet, dass** die erste Modellierung des Bayesschen Netzes (RB) für mindestens einen Knoten des Netzes eine Modellierung von mindestens einer Verbindung zwischen zwei Faktoren einer der vier Faktorengruppen ($F_1$, $F_2$, $F_3$, $F_4$) aufweist, wobei die Modellierung (RB) spezifiziert, ob die Faktoren unabhängig oder abhängig sind, wobei das Abhängigkeitsverhältnis zwischen mindestens zwei Faktoren durch einen Wert gemeinsamer bedingter Wahrscheinlichkeit einer Krankheit modelliert ist, wobei die vorhandenen Faktoren bekannt sind.

13. Verfahren nach einem der Ansprüche 9 bis 12, **dadurch gekennzeichnet, dass** es einen Schritt der Überprüfung des Vorhandenseins einer Verbindung zwischen erfassten Faktoren der verschiedenen Gruppen (ENS$_1$, ENS$_2$, ENS$_3$) in der Anzeichen- oder Krankheitsdatenbank umfasst, gegebenenfalls umfasst das Verfahren einen Schritt der Auswahl des Wertes der gemeinsamen Wahrscheinlichkeit, der den verknüpften Faktoren zugeordnet ist.

14. Verfahren nach einem der Ansprüche 9 bis 13, **dadurch gekennzeichnet, dass** es eine vierte Erfassung (ACQ$_4$) vierter Faktoren (F$_4$) umfasst, die ein bestimmtes Medizinprodukt (PROD$_1$) und mindestens ein zweites Anzeichen (S$_2$) beschreiben, das diesem bestimmten Medizinprodukt (PROD$_1$) zugeordnet ist, wobei das zweite Anzeichen (S$_2$) eine erste Sensitivitätsstatistik (SE$_i$,) und eine zweite Spezifitätsstatistik (SPi) für jede Krankheit einer zweiten vordefinierten Krankheitsliste (LIST$_2$) aufweist, die diesem zweiten Zeichen (S$_2$) zugeordnet ist; wobei der Schritt der Generierung (GEN) der Gesamtheit der Wahrscheinlichkeiten die eingegebenen Daten der vierten Erfassung (ACQ$_4$) berücksichtigt.

15. Rechnerprogrammprodukt, das direkt in den internen Speicher eines digitalen Rechners ladbar ist, umfassend Softwarecodeabschnitte für die Ausführung der Schritte des Verfahrens nach einem der Ansprüche 9 bis 14, wenn das Programm auf einem Rechner ausgeführt wird.

**Claims**

1. System (1) comprising a calculator for the generation of a list of probabilities (LIST$_1$) associated with a list of diseases for a first patient (P$_1$), said system comprising an interface enabling:

   ▪ a first acquisition (ACQ$_1$) of a first set (ENS$_1$) of first factors (F$_1$) relative to the first patient (P$_1$) and storing said first factors (F$_1$) in a memory, said first factors (F$_1$) comprising:

      ◦ an age value (AGE$_1$),
      ◦ a gender value (GEN$_1$);

   ▪ a second acquisition (ACQ$_2$) of a second set (ENS$_2$) of second factors (F$_2$), notably describing at least one disease (ANT$_1$) or information specific to said patient (P$_1$) and storing said second factors (F$_2$) in a memory, said disease (ANT$_1$) being extracted from a first database (BD$_M$), each disease (ANT$_1$) being associated with a first prevalence statistic (PR$_1$) and/or a first incidence statistic (IN$_1$), and each disease being associated with a list of signs (S$_k$), at least one sign corresponding to a symptom of a disease;
   ▪ a third acquisition (ACQ$_3$) of a third set (ENS$_3$) of third factors (F$_3$) describing at least one sign (S$_1$) and storing said third factors (F$_3$) in a memory of the system, said first sign (S$_1$) comprising a first sensitivity statistic (SEi) and a second specificity statistic (SPi) for each disease of a predefined list (LIST$_1$) of diseases (Mi) associated with said sign (S$_1$); sensitivity being modeled according to a scale of predefined values, each predefined value corresponding to a value of a sign sensitivity statistic for a given disease, specificity being modeled according to a scale of predefined values, each predefined value of the scale corresponding to a value of a sign specificity statistic for a given disease;
   said calculator generating, from a first modelling of a Bayesian network (RB) and input data comprising the data of the first, second and third acquisitions (ACQ$_1$, ACQ$_2$, ACQ$_3$), a set of probabilities, each probability being associated with a given disease of the first list (LIST$_1$);
   said system (1) comprising a graphic interface for displaying said first generated list (LIST$_1$).

2. System according to claim 1, **characterized in that** it comprises an interface enabling a fourth acquisition (ACQ$_4$) of fourth factors (F$_4$) describing a given medical product (PROD$_1$) and at least one second sign (S$_2$) associated with said given medical product (PROD$_1$), said second sign (S$_2$) comprising a first sensitivity statistic (SE$_i$,) and a second specificity statistic (SPi) for each disease of a second predefined list (LIST$_2$) of diseases associated with said second sign (S$_2$); the calculator generating the set of probabilities while taking into account as input the data of the fourth acquisition (ACQ$_4$) to generate the first list (LIST$_1$).

3. System (1) according to claim 2, **characterized in that** it comprises a memory for saving data describing at least two medical products (PROD$_1$, PROD$_2$) and saving data coming from a plurality of data acquisitions derived from a set of patients (P$_j$) in such a way that a first group of patients (GR$_1$) is associated with the first product (PROD$_1$) and a second group of patients (GR$_2$) is associated with the second product (PROD$_2$), the calculator generating two lists of conditional probabilities, each probability being associated with a given disease, the calculator performing a calculation to compare the two lists in order to deduce the presence of at least one difference in probabilities for a same disease between the two lists when said difference is above a predefined threshold.

4. System (1) according to any one of claims 1 to 3, **characterized in that** it comprises a linguistic resource comprising an ontology, a dictionary or a synonyms database making it possible to associate terms describing signs or diseases in the database (BDM, BDS) with a predefined textual corpus.

5. System (1) according to any one of claims 1 to 4, **characterized in that** each third factor ($F_3$) comprises a plurality of properties describing a sign and stored in a memory of the system, each property being associated with a sensitivity and specificity value.

6. System (1) according to any one of claims 1 to 5, **characterized in that** the first modelling of the Bayesian network (RB) comprises, for at least one node of the network, a modelling of at least one relationship between two factors of one of the four sets of factors ($F_1$, $F_2$, $F_3$, $F_4$), said modelling specifying if the factors are independent or dependent and being stored in a memory of the system.

7. System (1) according to claim 6, **characterized in that**:

   - when no dependency relationship between at least two factors present in the acquired data (ACQ1, $ACQ_2$, $ACQ_3$ $ACQ_4$) is specified, the calculation of the probability of a disease comprises a calculation of conditional probability ($P(M| F_i, F_k)$) from factors considered as independent;
   - when said Bayesian modelling specifies a dependency relationship between at least two factors ($F_i$, $F_k$), the probability of a disease comprises a selection in the database either of the joint conditional probability or a selection of a sensitivity and/or specificity value of the factors considered jointly.

8. System (1) according to any one of claims 2 to 7, **characterized in that** it comprises a calculator to establish a comparison between the data acquired (ACQ1, $ACQ_2$, $ACQ_3$ $ACQ_4$) through the interface and the data stored in the memory such that when an acquired risk factor ($F_2$) is associated with a prevalence (PR) or incidence (IN) statistic of a disease (M), the probability of the associated disease (P(M)) is initialized at the value stored in the database.

9. Method for generating a list of probabilities ($LIST_1$) associated with a list of diseases for a first patient ($P_1$), said method comprising the use of an interface for:

   - a first acquisition ($ACQ_1$) of a first set of first factors ($F_1$) relative to the first patient ($P_1$) comprising:

     ◦ an age value ($AGE_1$),
     ◦ a gender value ($GEN_1$);

   - a second acquisition ($ACQ_2$) of a second set ($ENS_2$) of second factors ($F_2$), called risk factors, notably describing at least one disease ($ANT_1$) or information specific to said patient ($P_1$), said disease ($ANT_1$) being extracted from a first database ($BD_M$), each disease ($ANT_1$) being associated with a first prevalence statistic ($PR_1$) and a first incidence statistic ($IN_1$), and each disease being associated with a list of signs;
   - a third acquisition ($ACQ_3$) of a third set ($ENS_3$) of third factors ($F_3$) describing at least one sign ($S_1$), said first sign ($S_1$) comprising a first sensitivity statistic ($SE_i$,) and a second specificity statistic (SPi) for each disease of a predefined list ($LIST_1$) of diseases (Mi) associated with said sign ($S_1$);

   said method further comprising the use of a calculator for the application of a first modelling of a Bayesian network (RB) with input data comprising the data of the first, second and third acquisitions ($ACQ_1$, $ACQ_2$, $ACQ_3$), for the generation of a set of probabilities, each probability being associated with a given disease of the first list ($LIST_1$).

10. Method according to claim 9, **characterized in that** the probability associated with a disease of the first list ($LIST_1$) is a conditional probability of a disease with the occurrence of a set of factors ($F_1$, $F_2$, $F_3$) comprising at least three elements from among the following list:

   - a predefined sign ($S_k$);
   - a predefined medical history ($ANT_k$);
   - a risk factor ($F_2$);

- a predefined age ($AGE_k$);
- a predefined gender ($GEN_k$);
- a predefined geographic location ($GEO_k$);

11. Method according to any one of claims 9 to 10, **characterized in that** at least one factor ($F_2$) of the second data acquisition ($ACQ_2$) is associated with a medical history (ANT1) and comprises at least one of the following criteria:

- a first date of appearance and/or;
- a frequency of appearance and/or;
- a genetic information.

12. Method according to any one of claims 9 to 11, **characterized in that** the first modelling of the Bayesian network (RB) comprises, for at least one node of the network, a modelling of at least one relationship between two factors of one of the four sets of factors ($F_1$, $F_2$, $F_3$, $F_4$), said modelling (RB) specifying if the factors are independent or dependent, the dependency relationship between at least two factors being modelled by a value of joint conditional probability of a disease knowing the factors present.

13. Method according to any one of claims 9 to 12, **characterized in that** it comprises a step of verifying the existence of a relationship between factors acquired from the different sets ($ENS_1$, $ENS_2$, $ENS_3$) in the database of signs or diseases, if need be, the method comprises a step of selecting the value of the joint probability associated with the linked factors.

14. Method according to any one of claims 9 to 13, **characterized in that** it comprises a fourth acquisition ($ACQ_4$) of fourth factors ($F_4$) describing a given medical product ($PROD_1$) and at least one second sign ($S_2$) associated with said given medical product ($PROD_1$), said second sign ($S_2$) comprising a first sensitivity statistic ($SE_i$,) and a second specificity statistic (SPi) for each disease of a second predefined list ($LIST_2$) of diseases associated with said second sign ($S_2$); the step of generating (GEN) the set of probabilities taking into account as input the data of the fourth acquisition ($ACQ_4$).

15. Computer program product directly loadable in the internal memory of a digital computer, comprising software code portions for the execution of the steps of the method according to any one of claims 9 to 14 when said program is executed on a computer.

FIG.1

FIG.2

FIG.3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 7720779 B

**Littérature non-brevet citée dans la description**

- **SEIXAS FLÁVIO LUIZ et al.** A Bayesian network décision model for supporting the diagnosis of dementia, Alzheimer's disease and mild cognitive impai. *COMPUTERS IN BIOLOGY AND MEDICINE, NEW YORK, NY, US,* 28 Mai 2014, vol. 51, 140-158